# EUROPEAN PATENT SPECIFICATION

(11) **EP 4 532 482 B1**
(45) Date of publication and mention of the grant of the patent: **04.03.2026**
(21) Application number: 23729094.5
(22) Date of filing: 26.05.2023
(51) Int. Cl.: C07D 401/04, A01N 43/56, A01N 43/653, A01P 13/00

(54) **HERBICIDAL DERIVATIVES**
HERBIZIDE DERIVATE
DÉRIVÉS HERBICIDES

(30) Priority: 01.06.2022 EP 22176865
(43) Date of publication of application: 09.04.2025
(73) Proprietor: Syngenta Crop Protection AG, 4058 Basel (CH)
(72) Inventor: MORRIS, James Alan, Bracknell Berkshire RG42 6EY (GB); MARTIN, Christopher, James, Bracknell Berkshire RG42 6EY (GB); WHALLEY, Louisa, Bracknell Berkshire RG42 6EY (GB)
(74) Representative: SYNGENTA IP
(86) International application number: PCT/EP2023/064172
(87) International publication number: WO 2023/232673

(56) References cited:
- EP-A1- 0 040 082
- EP-A1- 0 216 166
- EP-A2- 0 239 391
- WO-A1-2021/009229
- DE-A1- 3 210 598
- GB-A- 2 029 403
- US-A- 4 451 282
- US-A- 4 670 044

## Description

The present invention relates to herbicidal pyridone derivatives, e.g., as active ingredients, which have herbicidal activity. The invention also relates to agrochemical compositions which comprise at least one of the pyridone derivatives, to processes of preparation of these compounds and to uses of the pyridone derivatives or compositions in agriculture or horticulture for controlling weeds, in particular in crops of useful plants.

EP0239391, EP0127313, EP0040082, and GB2182931 describe pyridone derivatives as herbicidal agents.

According to the present invention, there is provided a compound of Formula (I): 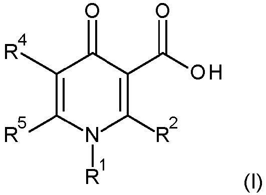 wherein
R¹ is C₁-C₆alkyl, C₁-C₆alkoxy, C₂-C₆alkenyl, C₂-C₆alkynyl, C₁-C₆alkoxyC₁-C₆alkyl, or C₃-C₆cycloalkyl;
R² is phenyl or heteroaryl, wherein the heteroaryl moiety is a 5- or 6-membered aromatic ring which comprises 1, 2, 3 or 4 heteroatoms individually selected from **N,** O and S, and wherein each phenyl and heteroaryl moiety may be optionally substituted with 1, 2, 3, or 4 groups, which may be the same or different, represented by R⁷;
R⁴ is heteroaryl wherein the heteroaryl moiety is a 5-membered aromatic monocyclic ring comprising 1, 2, or 3 nitrogen atoms, and wherein the heteroaryl moieties are attached to the rest of the molecule through a nitrogen atom in the heteroaryl ring, and wherein the heteroaryl moieties may each be optionally substituted with 1, 2, 3, or 4 groups, which may be the same or different, represented by R⁹;
R⁵ is hydrogen, halogen, C₁-C₆alkyl, C₁-C₆haloalkyl, or C₃-C₆cycloalkyl;
R⁷ is cyano, nitro, halogen, C₁-C₆alkyl, C₁-C₆alkoxy, C₁-C₆haloalkyl, C₁-C₆haloalkoxy, C₁-C₆alkoxyC₁-C₆alkyl, C₁-C₆alkylsulfanyl, C₁-C₆alkylsulfinyl, C₁-C₆alkylsulfonyl, C₁-C₆alkylsulfonamido, C₁-C₆alkylcarbonyl, C₁-C₆alkoxycarbonyl, C₁-C₆alkylaminocarbonyl, C₃-C₆cycloalkyl, C₃-C₆cycloalkylaminocarbonyl, or N,N-di(C₁-C₄alkyl)aminocarbonyl;
R⁹ is formyl, cyano, nitro, hydroxy, halogen, C₁-C₆alkyl, C₁-C₆alkoxy, C₁-C₆haloalkyl, C₁-C₆haloalkoxy, C₂-C₆alkenyloxy, cyanoC₁-C₆alkoxy, C₁-C₆alkoxyC₁-C₆alkyl, C₁-C₆alkoxyC₁-C₆alkoxy, C₁-C₆alkoxycarbonylC₁-C₆alkoxy, C₁-C₆alkoxycarbonyl, C₁-C₆alkylsulfanyl, C₁-C₆alkylsulfinyl, C₁-C₆alkylsulfonyl, C₁-C₆alkylsulfonamido, C₁-C₆alkylcarbonyl, C₁-C₆alkylaminocarbonyl, C₁-C₆alkylcarbonylamino, C₁-C₆alkylamino, C₁-C₆alkoxyC₁-C₆alkylaminocarbonyl, C₁-C₆alkoxyC₁-C₆alkylcarbonylamino, C₂-C₆alkenylcarbonylamino, C₃-C₆cycloalkyl, C₃-C₆cycloalkylaminocarbonyl, N,N-di(C₁-C₄alkyl)amino, N,N-di(C₁-C₄alkyl)aminocarbonyl, or benzyloxy;
wherein any two adjacent R⁹ groups together with the carbon atoms to which they are attached, may form a C₃-C₆cycloalkyl ring or phenyl ring, wherein the C₃-C₆cycloalkyl and phenyl moieties may be optionally substituted with 1, 2, 3 or 4 groups, which may be the same or different, represented by R¹⁰; or
any two adjacent R⁹ groups together with the carbon atoms to which they are attached, may form a 5- or 6-membered heteroaryl ring, wherein the heteroaryl moiety is a 5- or 6-membered aromatic ring which comprises 1, 2, 3 or 4 heteroatoms individually selected from N, O and S, and wherein the heteroaryl moiety may be optionally substituted with 1, 2, 3 or 4 groups, which may be the same or different, represented by R¹⁰; or
any two adjacent R⁹ groups together with the carbon atoms to which they are attached, may form a 5- or 6-membered heterocyclyl ring, wherein the heterocyclyl moiety is a 5- or 6-membered comprising 1 or 2 heteroatoms selected from O and N, and wherein the heterocyclyl ring may be optionally substituted with 1, 2, 3 or 4 groups, which may be the same or different, represented by R¹⁰;
R¹⁰ is halogen, C₁-C₃alkyl, or C₁-C₃alkoxy;
or a salt or an N-oxide thereof.

Surprisingly, it has been found that the novel compounds of Formula (I) have, for practical purposes, a very advantageous level of herbicidal activity.

According to a second aspect of the invention, there is provided an agrochemical composition comprising a herbicidally effective amount of a compound of Formula (I) according to the present invention. Such an agricultural composition may further comprise at least one additional active ingredient and/or an agrochemically-acceptable diluent or carrier.

According to a third aspect of the invention, there is provided a method of controlling weeds at a locus comprising applying to the locus a weed controlling amount of a composition comprising a compound of Formula (I).

According to a fourth aspect of the invention, there is provided the use of a compound of Formula (I) as a herbicide.

Where substituents are indicated as being "optionally substituted", this means that they may or may not carry one or more identical or different substituents, e.g., one, two or three R⁹ substituents. For example, C₁-C₆alkyl substituted by 1, 2 or 3 halogens, may include, but not be limited to, -CH₂Cl, -CHCl₂, -CCl₃, -CH₂F, -CHF₂, -CF₃, -CH₂CF₃ or -CF₂CH₃ groups. As another example, C₁-C₆alkoxy substituted by 1, 2 or 3 halogens, may include, but not limited to, CH₂ClO-, CHCl₂O-, CCl₃O-, CH₂FO-, CHF₂O-, CF₃O-, CF₃CH₂O- or CH₃CF₂O- groups.

As used herein, the term "cyano" means a -CN group.

As used herein, the term "hydroxy" or "hydroxyl" refers to an -OH group.

As used herein, the term "halogen" refers to fluorine (fluoro), chlorine (chloro), bromine (bromo) or iodine (iodo).

As used herein, the term "nitro" means an -NO₂ group.

As used herein, the term "formyl" means a -C(O)H group.

As used herein, the term "acetyl" means a -C(O)CH₃ group.

As used herein, the term "C₁-C₆alkyl" refers to a straight or branched hydrocarbon chain radical consisting solely of carbon and hydrogen atoms, containing no unsaturation, having from one to six carbon atoms, and which is attached to the rest of the molecule by a single bond. "C₁-C₄alkyl" and "C₁-C₃alkyl" are to be construed accordingly. Examples of C₁-C₆alkyl include, but are not limited to, methyl, ethyl, n-propyl, and the isomers thereof, for example, iso-propyl. A "C₁-C₆alkylene" group refers to the corresponding definition of C₁-C₆alkyl, except that such radical is attached to the rest of the molecule by two single bonds. The term "C₁-C₂alkylene" is to be construed accordingly. Examples of C₁-C₆alkylene, include, but are not limited to, -CH₂-, -CH₂CH₂- and -(CH₂)₃-.

As used herein, the term "C₁-C₆haloalkyl" refers a C₁-C₆alkyl radical as generally defined above substituted by one or more of the same or different halogen atoms. The terms "C₁-C₄haloalkyl" and "C₁-C₃haloalkyl", are to be construed accordingly. Examples of C₁-C₆haloalkyl include, but are not limited to trifluoromethyl.

As used herein, the term "C₁-C₆alkoxy" refers to a radical of the formula -ORₐ where Rₐ is a C₁-C₆alkyl radical as generally defined above. The terms "C₁-C₄alkoxy" and "C₁-C₃alkoxy" are to be construed accordingly. Examples of C₁-C₆alkoxy include, but are not limited to, methoxy, ethoxy, 1-methylethoxy (iso-propoxy), and propoxy.

As used herein, the term "C₁-C₆haloalkoxy" refers to a C₁-C₆alkoxy radical as generally defined above substituted by one or more of the same or different halogen atoms. The terms "C₁-C₄haloalkoxy" and "C₁-C₃haloalkoxy", are to be construed accordingly. Examples of C₁-C₆haloalkoxy include, but are not limited to trifluoromethoxy.

As used herein, the term "cyanoC₁-C₆alkoxy" refers to a C₁-C₆alkoxy radical as generally defined above substituted by one or more cyano groups. The terms "cyanoC₁-C₄alkoxy" and "cyanoC₁-C₃alkoxy", are to be construed accordingly. Examples of cyanoC₁-C₆alkoxy include, but are not limited to cyanoethoxy.

As used herein, the term "C₂-C₆alkenyl" refers to a straight or branched hydrocarbon chain radical group consisting solely of carbon and hydrogen atoms, containing at least one double bond that can be of either the (E)- or (Z)-configuration, having from two to six carbon atoms, which is attached to the rest of the molecule by a single bond. The term "C₂-C₃alkenyl" is to be construed accordingly. Examples of C₂-C₆alkenyl include, but are not limited to, ethenyl (vinyl), prop-1-enyl, prop-2-enyl (allyl), but-1-enyl.

As used herein, the term "C₂-C₆alkynyl" refers to a straight or branched hydrocarbon chain radical group consisting solely of carbon and hydrogen atoms, containing at least one triple bond, having from two to six carbon atoms, and which is attached to the rest of the molecule by a single bond. The term "C₂-C₃alkynyl" is to be construed accordingly. Examples of C₂-C₆alkynyl include, but are not limited to, ethynyl, prop-1-ynyl, but-1-ynyl.

As used herein, the term "C₂-C₆alkenyloxy" refers to a straight or branched hydrocarbon chain radical group consisting solely of carbon and hydrogen atoms, containing at least one double bond that can be of either the (E)- or (Z)-configuration, having from two to six carbon atoms, which is attached to the rest of the molecule through an oxygen linker.

As used herein, the term "C₁-C₆alkoxyC₁-C₆alkyl" refers to a radical of the formula R_{b}ORₐ- wherein R_{b} is a C₁-C₆alkyl radical as generally defined above, and Rₐ is a C₁-C₆alkylene radical as generally defined above. The term "C₁-C₄alkoxyC₁-C₄alkyl" is to be construed accordingly.

As used herein, the term "C₁-C₆alkoxyC₁-C₆alkoxy" refers to a radical of the formula R_{b}ORₐO-wherein Rₐ and R_{b} are both C₁-C₆alkylene radicals as generally defined above. The term "C₁-C₄alkoxyC₁-C₄alkoxy" is to be construed accordingly.

As used herein, the term "C₁-C₆alkoxycarbonylC₁-C₆alkoxy" refers to a radical of the formula R_{b}OC(O)RₐO-, wherein Rₐ and R_{b} are both C₁-C₆alkylene radicals as generally defined above.

As used herein, the term "C₃-C₆cycloalkyl" refers to a radical which is a monocyclic saturated ring system and which contains 3 to 6 carbon atoms. The terms "C₃-C₅cycloalkyl" and "C₃-C₄cycloalkyl" are to be construed accordingly. Examples of C₃-C₆cycloalkyl include, but are not limited to, cyclopropyl, cyclobutyl, cyclopentyl, and cyclohexyl.

As used herein, the term "C₃-C₆cycloalkylaminocarbonyl" refers to a C₃-C₆cycloalkyl ring attached to the rest of the molecule through an -NHC(O)- linker.

As used herein, the term "benzyloxy" refers to a benzyl ring attached to the rest of the molecule through an oxygen atom.

Unless described otherwise, as used herein, the term "heteroaryl" refers to a 5- or 6-membered aromatic monocyclic ring radical which comprises 1, 2, 3 or 4 heteroatoms individually selected from nitrogen, oxygen and sulfur. In some instances the heteroaryl moieties may be attached to the rest of the molecule through a nitrogen atom in the heteroaryl ring. In other instances, the heteroaryl moieties may be attached to the rest of the molecule through a carbon atom in the heteroaryl ring. Examples of heteroaryl include, but are not limited to, furanyl, pyrrolyl, thienyl, pyrazolyl, imidazolyl, thiazolyl, isothiazolyl, oxazolyl, isoxazolyl, triazolyl, tetrazolyl, pyrazinyl, pyridazinyl, pyrimidyl or pyridyl.

As used herein, the term "heterocyclyl" refers to a stable 4-, 5- or 6-membered non-aromatic monocyclic ring which comprises 1, 2 or 3 heteroatoms, wherein the heteroatoms are individually selected from nitrogen, oxygen and sulfur. The heterocyclyl radical may be bonded to the rest of the molecule via a carbon atom or heteroatom. Examples of heterocyclyl include, but are not limited to, aziridinyl, azetidinyl, oxetanyl, thietanyl, tetrahydrofuryl, pyrrolidinyl, pyrazolidinyl, imidazolidnyl, piperidinyl, piperazinyl, morpholinyl, dioxolanyl, dithiolanyl and thiazolidinyl.

As used herein, the term "C₁-C₆alkylcarbonyl" refers to a radical of the formula -C(O)Rₐ, where Rₐ is a C₁-C₆alkyl radical as generally defined above. Examples of C₁-C₆alkylcarbonyl include, but are not limited to, acetyl.

As used herein, the term "C₁-C₆alkoxycarbonyl" refers to a radical of the formula -C(O)ORₐ, where Rₐ is a C₁-C₆alkyl radical as generally defined above.

As used herein, the term "C₁-C₆alkylamino" refers to a radical of the formula RₐNH- wherein Rₐ is a C₁-C₆alkyl radical as generally defined above.

As used herein, the term "C₁-C₆alkylaminocarbonyl" refers to a radical of the formula -C(O)NHRₐ, wherein Rₐ is a C₁-C₆alkyl radical as generally defined above.

As used herein, the term "C₁-C₆alkylcarbonylamino" refers to a radical of the formula RₐC(O)NH- wherein Rₐ is a C₁-C₆alkyl radical as generally defined above.

As used herein, the term "C₁-C₆alkoxyC₁-C₆alkylaminocarbonyl" refers to a radical of the formula RₐOR_{b}NHC(O)-, wherein Rₐ is a C₁-C₆alkyl radical, Rₐ is a C₁-C₆alkylene radical as generally defined above.

As used herein, the term "C₁-C₆alkoxyC₁-C₆alkylcarbonylamino" refers to a radical of the formula RₐOR_{b}C(O)NH-, wherein Rₐ is a C₁-C₆alkyl radical, Rₐ is a C₁-C₆alkylene radical as generally defined above.

As used herein, the term "C₂-C₆alkenylcarbonylamino" refers to a radical of the formula RₐOC(O)NH-, wherein Rₐ is a C₁-C₆alkylene radical as generally defined above.

As used herein, the term "N,N-di(C₁-C₄alkyl)amino" refers to a radical of the formula (Rₐ)(R_{b})NH- , wherein Rₐ and R_{b} are each individually a C₁-C₄alkyl radical as generally defined above.

As used herein, the term "N,N-di(C₁-C₄alkyl)aminocarbonyl" refers to a radical of the formula - C(O)N(Rₐ)(R_{b}), wherein Rₐ and R_{b} are each individually a C₁-C₄alkyl radical as generally defined above. The term "N,N-di(C₁-C₃alkyl)aminocarbonyl" is to be construed accordingly.

As used herein, the term "C₁-C₆alkylsulfanyl" refers to a radical of the formula -SRₐ, where Rₐ is a C₁-C₆alkyl radical as generally defined above. The terms "C₁-C₄alkylsulfanyl" and "C₁-C₃alkylsulfanyl", are to be construed accordingly. Examples of C₁-C₆alkylsulfanyl include, but are not limited to methylsulfanyl.

As used herein, the term "C₁-C₆alkylsulfinyl" refers to a radical of the formula -S(O)Rₐ, where Rₐ is a C₁-C₆alkyl radical as generally defined above. The terms "C₁-C₄alkylsulfinyl" and "C₁-C₃alkylsulfinyl", are to be construed accordingly. Examples of C₁-C₆alkylsulfinyl include, but are not limited to methylsulfinyl.

As used herein, the term "C₁-C₆alkylsulfonyl" refers to a radical of the formula -S(O)₂Rₐ, where Rₐ is a C₁-C₆alkyl radical as generally defined above. The terms "C₁-C₄alkylsulfonyl" and "C₁-C₃alkylsulfonyl", are to be construed accordingly. Examples of C₁-C₆alkylsolfanyl include, but are not limited to methylsulfonyl.

As used herein, the term "C₁-C₆alkylsulfonamido" refers to a radical of the formula -NHS(O)₂Rₐ, wherein Rₐ is a C₁-C₆alkyl radical as generally defined above.

The presence of one or more possible stereogenic elements in a compound of formula (I) means that the compounds may occur in optically isomeric forms, i.e., enantiomeric or diastereomeric forms. Also, atropisomers may occur as a result of restricted rotation about a single bond. Formula (I) is intended to include all those possible isomeric forms and mixtures thereof. The present invention includes all those possible isomeric forms and mixtures thereof for a compound of formula (I). Likewise, formula (I) is intended to include all possible tautomers. The present invention includes all possible tautomeric forms for a compound of formula (I).

In each case, the compounds of formula (I) according to the invention are in free form, in oxidized form as an N-oxide, or in salt form, e.g., an agronomically usable salt form. Salts that the compounds of Formula (I) may form with amines, including primary, secondary and tertiary amines (for example ammonia, dimethylamine and triethylamine), alkali metal and alkaline earth metal bases, transition metals or quaternary ammonium bases are preferred.

N-oxides are oxidized forms of tertiary amines or oxidized forms of nitrogen-containing heteroaromatic compounds. They are described for instance in the book "Heterocyclic N-oxides" by A. Albini and S. Pietra, CRC Press, Boca Raton (1991).

The following list provides definitions, including preferred definitions, for substituents R¹, R², R⁴, R⁵, R⁶, R⁷, R⁹, R¹⁰ and with reference to compounds of Formula (I). For any one of these substituents, any of the definitions given below may be combined with any definition of any other substituent given below or elsewhere in this document.

R¹ is C₁-C₆alkyl, C₁-C₆alkoxy, C₂-C₆alkenyl, C₂-C₆alkynyl, C₁-C₆alkoxyC₁-C₆alkyl, or C₃-C₆cycloalkyl. Preferably, R' is C₁-C₄alkyl, C₁-C₄alkoxy, C₂-C₄alkenyl, C₂-C₄alkynyl, C₁-C₄alkoxyC₁-C₄alkyl, or C₃-C₆cycloalkyl. More preferably, R' is C₁-C₃alkyl, C₁-C₃alkoxy, C₂-C₃alkenyl, C₂-C₃alkynyl, C₁-C₃alkoxyC₁-C₃alkyl, or C₃-C₄cycloalkyl. More preferably still, R¹ is methyl, ethyl, n-propyl, methoxy, 2-methoxyethyl, ally, and prop-2-ynyl. Even more preferably, R¹ is methyl or ethyl. In one set of embodiments, R¹ is ethyl.

R² is phenyl or heteroaryl, wherein the heteroaryl moiety is a 5- or 6-membered aromatic ring which comprises 1, 2, 3 or 4 heteroatoms individually selected from N, O and S, and wherein each phenyl and heteroaryl moiety may be optionally substituted with 1, 2, 3, or 4 groups, which may be the same or different, represented by R⁷.

Preferably, R² is phenyl or heteroaryl, wherein the heteroaryl moiety is a 5- or 6-membered aromatic ring which comprises 1, 2, or 3 heteroatoms individually selected from N, O and S, and wherein each phenyl and heteroaryl moiety may be optionally substituted with 1, 2, or 3 groups, which may be the same or different, represented by R⁷.

More preferably, R² is phenyl or heteroaryl, wherein the heteroaryl moiety is a 5- or 6-membered aromatic ring which comprises 1 or 2 heteroatoms individually selected from N and O, and wherein each phenyl and heteroaryl moiety may be optionally substituted with 1, 2, or 3 groups, which may be the same or different, represented by R⁷.

More preferably still, R² is phenyl or heteroaryl, wherein the heteroaryl moiety is a 5- or 6-membered aromatic ring which comprises 1 or 2 heteroatoms individually selected from N and O, and wherein each phenyl and heteroaryl moiety may be optionally substituted with 1 or 2 groups, which may be the same or different, represented by R⁷.

Even more preferably, R² is phenyl optionally substituted with 1 or 2 groups, which may be the same or different, represented by R⁷. In one set of embodiments, R² is 3,4-dichlorophenyl or 3,4-difluorophenyl, preferably 3,4-dichlorophenyl.

R⁴ is heteroaryl wherein the heteroaryl moiety is a 5-membered aromatic monocyclic ring comprising 1, 2, or 3 nitrogen atoms, and wherein the heteroaryl moieties are attached to the rest of the molecule through a nitrogen atom in the heteroaryl ring, and wherein the heteroaryl moieties may each be optionally substituted with 1, 2, 3, or 4 groups, which may be the same or different, represented by R⁹.

Preferably, R⁴ is heteroaryl wherein the heteroaryl moiety is a 5-membered aromatic monocyclic ring comprising 1, 2, or 3 nitrogen atoms, and wherein the heteroaryl moieties are attached to the rest of the molecule through a nitrogen atom in the heteroaryl ring, and wherein the heteroaryl moieties may each be optionally substituted with 1, 2, or 3 groups, which may be the same or different, represented by R⁹.

More preferably, R⁴ is heteroaryl wherein the heteroaryl moiety is a 5-membered aromatic monocyclic ring comprising 2 or 3 nitrogen atoms, and wherein the heteroaryl moieties are attached to the rest of the molecule through a nitrogen atom in the heteroaryl ring, and wherein the heteroaryl moieties may each be optionally substituted with 1, 2, or 3 groups, which may be the same or different, represented by R⁹.

More preferably still, R⁴ is pyrazolyl, imidazolyl, triazolyl, wherein any of the pyrazolyl, imidazolyl, and triazolyl moieties may each be optionally substituted with 1, 2, or 3 groups, which may be the same or different, represented by R⁹.

In one set of embodiments, R⁴ is heteroaryl wherein the heteroaryl moiety is a 5-membered aromatic monocyclic ring comprising 2 nitrogen atoms, and wherein the heteroaryl moieties are attached to the rest of the molecule through a nitrogen atom in the heteroaryl ring, and wherein the heteroaryl ring is optionally substituted with 3 groups represented by R⁹, wherein one R⁹ group is selected from formyl, cyano, nitro, hydroxy, halogen, C₁-C₆alkyl, C₁-C₆alkoxy, C₁-C₆haloalkyl, C₁-C₆haloalkoxy, C₂-C₆alkenyloxy, cyanoC₁-C₆alkoxy, C₁-C₆alkoxyC₁-C₆alkyl, C₁-C₆alkoxyC₁-C₆alkoxy, C₁-C₆alkoxycarbonylC₁-C₆alkoxy, C₁-C₆alkoxycarbonyl, C₁-C₆alkylsulfanyl, C₁-C₆alkylsulfinyl, C₁-C₆alkylsulfonyl, C₁-C₆alkylsulfonamido, C₁-C₆alkylcarbonyl, C₁-C₆alkylaminocarbonyl, C₁-C₆alkylcarbonylamino, C₁-C₆alkylamino, C₁-C₆alkoxyC₁-C₆alkylaminocarbonyl, C₁-C₆alkoxyC₁-C₆alkylcarbonylamino, C₂-C₆alkenylcarbonylamino, C₃-C₆cycloalkyl, C₃-C₆cycloalkylaminocarbonyl, N,N-di(C₁-C₄alkyl)amino, N,N-di(C₁-C₄alkyl)aminocarbonyl, or benzyloxy, and wherein any two adjacent R⁹ groups together with the carbon atoms to which they are attached, may form a phenyl ring, wherein the resulting phenyl moiety may be optionally substituted with a single R¹⁰ group.

In another set of embodiments, R⁴ is pyrazolyl, triazolyl, or indazolyl, wherein each moiety is optionally substituted by one or two substituents selected from cyano, halogen, C₁-C₃alkyl, C₁-C₃haloalkyl, C₁-C₃alkoxy, C₁-C₃haloalkoxy, C₁-C₂alkoxyC₁-C₂alkyl, C₁-C₂alkylcarbonyl, and C₃-C₄cycloalkyl. Preferably, R⁴ is pyrazolyl optionally substituted by one or two substituents selected from cyano, halogen, C₁-C₃alkyl, C₁-C₃haloalkyl, C₁-C₃alkoxy, C₁-C₃haloalkoxy, C₁-C₂alkoxyC₁-C₂alkyl, C₁-C₂alkylcarbonyl, and C₃-C₄cycloalkyl. More preferably, R⁴ is pyrazolyl optionally substituted by a single substituent selected from halogen and C₁-C₃haloalkyl, preferably, chloro and trifluoromethyl.

In a particularly preferred set of embodiments, R⁴ is 3-(trifluoromethyl)pyrazol-1-yl, 3-chloropyrazol-1-yl, 4-chloropyrazol-1-yl, 3-methylpyrazol-1-yl, 3-methoxypyrazol-1-yl, 3-isopropoxypyrazol-1-yl, 3-cyclopropylpyrazol-1-yl, 3-fluoropyrazol-1-yl, 3-(methoxymethyl)pyrazol-1-yl, 3-acetylpyrazol-1-yl, 3-ethoxypyrazol-1-yl, 3-(trifluoromethyl)pyrazol-1-yl, 3-isopropylpyrazol-1-yl, 6-fluoro-3-methyl-indazol-1-yl, 4-methoxyindazol-1-yl, 5-fluoroindazol-1-yl, 2,2-difluoroethoxy)pyrazol-1-yl, 3-cyanopyrazol-1-yl, 6-methoxyindazol-1-yl, 7-fluoroindazol-2-yl, or 3-chloro-1,2,4-triazol-1-yl. An especially preferred set of embodiments is one in which R⁴ is 3-(trifluoromethyl)pyrazol-1-yl, 3-chloropyrazol-1-yl, or 4-chloropyrazol-1-yl.

R⁵ is hydrogen, halogen, C₁-C₆alkyl, C₁-C₆haloalkyl, or C₃-C₆cycloalkyl. Preferably, R⁵ is hydrogen, halogen, C₁-C₄alkyl, C₁-C₄haloalkyl, or C₃-C₆cycloalkyl. More preferably, R⁵ is hydrogen, halogen, C₁-C₃alkyl, C₁-C₃haloalkyl, or C₃-C₅cycloalkyl. Even more preferably, R⁵ is hydrogen, halogen, C₁-C₃alkyl, or C₃-C₄cycloalkyl. More preferably still, R⁵ is hydrogen or C₁-C₃alkyl. Even more preferably still, R⁵ is C₁-C₃alkyl, and preferably methyl.

R⁷ is cyano, nitro, halogen, C₁-C₆alkyl, C₁-C₆alkoxy, C₁-C₆haloalkyl, C₁-C₆haloalkoxy, C₁-C₆alkoxyC₁-C₆alkyl, C₁-C₆alkylsulfanyl, C₁-C₆alkylsulfinyl, C₁-C₆alkylsulfonyl, C₁-C₆alkylsulfonamido, C₁-C₆alkylcarbonyl, C₁-C₆alkoxycarbonyl, C₁-C₆alkylaminocarbonyl, C₃-C₆cycloalkyl, C₃-C₆cycloalkylaminocarbonyl, or N,N-di(C₁-C₄alkyl)aminocarbonyl.

Preferably, R⁷ is cyano, nitro, halogen, C₁-C₄alkyl, C₁-C₄alkoxy, C₁-C₄haloalkyl, C₁-C₄haloalkoxy, C₁-C₄alkoxyC₁-C₃alkyl, C₁-C₄alkylsulfanyl, C₁-C₄alkylsulfinyl, C₁-C₄alkylsulfonyl, C₁-C₄alkylsulfonamido, C₁-C₄alkylcarbonyl, C₁-C₄alkoxycarbonyl, C₁-C₄alkylaminocarbonyl, C₃-C₆cycloalkyl, C₃-C₆cycloalkylaminocarbonyl, or N,N-di(C₁-C₃alkyl)aminocarbonyl.

More preferably, R⁷ is cyano, nitro, halogen, C₁-C₃alkyl, C₁-C₃alkoxy, C₁-C₃haloalkyl, C₁-C₃haloalkoxy, C₁-C₃alkoxyC₁-C₃alkyl, C₁-C₃alkylsulfanyl, C₁-C₃alkylsulfinyl, C₁-C₃alkylsulfonyl, C₁-C₃alkylsulfonamido, C₁-C₃alkylcarbonyl, C₁-C₃alkoxycarbonyl, C₁-C₃alkylaminocarbonyl, C₃-C₆cycloalkyl, C₃-C₆cycloalkylaminocarbonyl, or N,N-di(C₁-C₃alkyl)aminocarbonyl.

More preferably still, R⁷ is cyano, nitro, halogen, C₁-C₃alkyl, C₁-C₃alkoxy, C₁-C₃haloalkyl, C₁-C₃haloalkoxy, C₁-C₃alkoxyC₁-C₃alkyl, C₁-C₃alkylcarbonyl, or C₃-C₆cycloalkyl. Even more preferably, R⁷ is cyano, nitro, chloro, fluoro, methyl, isopropyl, methoxy, ethoxy, isopropoxy, trifluoromethyl, trifluoromethoxy, difluoromethoxy, 2,2-difluoroethoxy, methoxymethyl, acetyl, or cyclopropyl.

In one set of embodiments, R⁷ is cyano, nitro, or halogen, preferably halogen, and more preferably chloro or fluoro.

R⁹ is formyl, cyano, nitro, hydroxy, halogen, C₁-C₆alkyl, C₁-C₆alkoxy, C₁-C₆haloalkyl, C₁-C₆haloalkoxy, C₂-C₆alkenyloxy, cyanoC₁-C₆alkoxy, C₁-C₆alkoxyC₁-C₆alkyl, C₁-C₆alkoxyC₁-C₆alkoxy, C₁-C₆alkoxycarbonylC₁-C₆alkoxy, C₁-C₆alkoxycarbonyl, C₁-C₆alkylsulfanyl, C₁-C₆alkylsulfinyl, C₁-C₆alkylsulfonyl, C₁-C₆alkylsulfonamido, C₁-C₆alkylcarbonyl, C₁-C₆alkylaminocarbonyl, C₁-C₆alkylcarbonylamino, C₁-C₆alkylamino, C₁-C₆alkoxyC₁-C₆alkylaminocarbonyl, C₁-C₆alkoxyC₁-C₆alkylcarbonylamino, C₂-C₆alkenylcarbonylamino, C₃-C₆cycloalkyl, C₃-C₆cycloalkylaminocarbonyl, N,N-di(C₁-C₄alkyl)amino, N,N-di(C₁-C₄alkyl)aminocarbonyl, or benzyloxy; or
any two adjacent R⁹ groups together with the carbon atoms to which they are attached, may form a C₃-C₆cycloalkyl ring or phenyl ring, wherein the C₃-C₆cycloalkyl and phenyl moieties may be optionally substituted with 1, 2, 3 or 4 groups, which may be the same or different, represented by R¹⁰; or
any two adjacent R⁹ groups together with the carbon atoms to which they are attached, may form a 5- or 6-membered heteroaryl ring, wherein the heteroaryl moiety is a 5- or 6-membered aromatic ring which comprises 1, 2, 3 or 4 heteroatoms individually selected from N, O and S, and wherein the heteroaryl moiety may be optionally substituted with 1, 2, 3 or 4 groups, which may be the same or different, represented by R¹⁰; or
any two adjacent R⁹ groups together with the carbon atoms to which they are attached, may form a 5- or 6-membered heterocyclyl ring, wherein the heterocyclyl moiety is a 5- or 6-membered comprising 1 or 2 heteroatoms selected from O and N, and wherein the heterocyclyl ring may be optionally substituted with 1, 2, 3 or 4 groups, which may be the same or different, represented by R¹⁰.

Preferably, R⁹ is formyl, cyano, nitro, hydroxy, halogen, C₁-C₆alkyl, C₁-C₆alkoxy, C₁-C₆haloalkyl, C₁-C₆haloalkoxy, C₂-C₆alkenyloxy, cyanoC₁-C₄alkoxy, C₁-C₄alkoxyC₁-C₄alkyl, C₁-C₆alkoxyC₁-C₆alkoxy, C₁-C₄alkoxycarbonylC₁-C₄alkoxy, C₁-C₆alkoxycarbonyl, C₁-C₄alkylsulfanyl, C₁-C₄alkylsulfinyl, C₁-C₄alkylsulfonyl, C₁-C₄alkylsulfonamido, C₁-C₆alkylcarbonyl, C₁-C₆alkylaminocarbonyl, C₁-C₆alkylcarbonylamino, C₁-C₆alkylamino, C₁-C₆alkoxyC₁-C₆alkylaminocarbonyl, C₁-C₄alkoxyC₁-C₄alkylcarbonylamino, C₂-C₆alkenylcarbonylamino, C₃-C₆cycloalkyl, C₃-C₆cycloalkylaminocarbonyl, N,N-di(C₁-C₃alkyl)amino, N,N-di(C₁-C₃alkyl)aminocarbonyl, or benzyloxy; or
any two adjacent R⁹ groups together with the carbon atoms to which they are attached, may form a C₃-C₆cycloalkyl ring or phenyl ring, wherein the C₃-C₆cycloalkyl and phenyl moieties may be optionally substituted with 1, 2, or 3 groups, which may be the same or different, represented by R¹⁰; or
any two adjacent R⁹ groups together with the carbon atoms to which they are attached, may form a 5- or 6-membered heteroaryl ring, wherein the heteroaryl moiety is a 5- or 6-membered aromatic ring which comprises 1, 2, or 3 heteroatoms individually selected from N, O and S, and wherein the heteroaryl moiety may be optionally substituted with 1, 2, or 3 groups, which may be the same or different, represented by R¹⁰; or
any two adjacent R⁹ groups together with the carbon atoms to which they are attached, may form a 5- or 6-membered heterocyclyl ring, wherein the heterocyclyl moiety is a 5- or 6-membered comprising 1 or 2 heteroatoms selected from O and N, and wherein the heterocyclyl ring may be optionally substituted with 1, 2, or 3 groups, which may be the same or different, represented by R¹⁰.

More preferably, R⁹ is formyl, cyano, nitro, hydroxy, halogen, C₁-C₄alkyl, C₁-C₄alkoxy, C₁-C₄haloalkyl, C₁-C₄haloalkoxy, C₂-C₄alkenyloxy, cyanoC₁-C₃alkoxy, C₁-C₃alkoxyC₁-C₃alkyl, C₁-C₃alkoxyC₁-C₃alkoxy, C₁-C₃alkoxycarbonylC₁-C₃alkoxy, C₁-C₃alkoxycarbonyl, C₁-C₃alkylsulfanyl, C₁-C₃alkylsulfinyl, C₁-C₃alkylsulfonyl, C₁-C₃alkylsulfonamido, C₁-C₃alkylcarbonyl, C₁-C₃alkylaminocarbonyl, C₁-C₃alkylcarbonylamino, C₁-C₃alkylamino, C₁-C₃alkoxyC₁-C₃alkylaminocarbonyl, C₁-C₃alkoxyC₁-C₃alkylcarbonylamino, C₂-C₄alkenylcarbonylamino, C₃-C₆cycloalkyl, C₃-C₆cycloalkylaminocarbonyl, N,N-di(C₁-C₃alkyl)amino, N,N-di(C₁-C₃alkyl)aminocarbonyl, or benzyloxy; or
any two adjacent R⁹ groups together with the carbon atoms to which they are attached, may form a C₃-C₆cycloalkyl ring or phenyl ring, wherein the C₃-C₆cycloalkyl and phenyl moieties may be optionally substituted with 1 or 2 groups, which may be the same or different, represented by R¹⁰; or
any two adjacent R⁹ groups together with the carbon atoms to which they are attached, may form a 5- or 6-membered heteroaryl ring, wherein the heteroaryl moiety is a 5- or 6-membered aromatic ring which comprises 1, 2, or 3 heteroatoms individually selected from N, O and S, and wherein the heteroaryl moiety may be optionally substituted with 1 or 2 groups, which may be the same or different, represented by R¹⁰; or
any two adjacent R⁹ groups together with the carbon atoms to which they are attached, may form a 5- or 6-membered heterocyclyl ring, wherein the heterocyclyl moiety is a 5- or 6-membered comprising 1 or 2 heteroatoms selected from O and N, and wherein the heterocyclyl ring may be optionally substituted with 1 or 2 groups, which may be the same or different, represented by R¹⁰.

Even more preferably, R⁹ is formyl, cyano, nitro, halogen, C₁-C₄alkyl, C₁-C₃alkoxy, C₁-C₃haloalkyl, C₁-C₂haloalkoxy, C₂-C₃alkenyloxy, cyanoC₁-C₂alkoxy, C₁-C₃alkoxyC₁-C₃alkyl, C₁-C₃alkoxyC₁-C₃alkoxy, C₁-C₃alkoxycarbonylC₁-C₃alkoxy, C₁-C₃alkoxycarbonyl, C₁-C₃alkylsulfanyl, C₁-C₃alkylsulfonyl, C₁-C₃alkylcarbonyl, C₁-C₃alkylaminocarbonyl, C₁-C₃alkylcarbonylamino, C₁-C₃alkylamino, C₁-C₃alkoxyC₁-C₃alkylaminocarbonyl, C₁-C₂alkoxyC₁-C₂alkylcarbonylamino, C₃-C₄alkenylcarbonylamino, C₃-C₄cycloalkyl, or N,N-di(C₁-C₃alkyl)amino; or
any two adjacent R⁹ groups together with the carbon atoms to which they are attached, may form a C₅-C₆cycloalkyl ring or phenyl ring, wherein the C₅-C₆cycloalkyl and phenyl moieties may be optionally substituted with a single R¹⁰ group; or
any two adjacent R⁹ groups together with the carbon atoms to which they are attached, may form a 6-membered heteroaryl ring, wherein the heteroaryl moiety is a 6-membered aromatic ring which comprises a single nitrogen atom, and wherein the heteroaryl moiety may be optionally substituted with a single R¹⁰ group; or
any two adjacent R⁹ groups together with the carbon atoms to which they are attached, may form a 5- or 6-membered heterocyclyl ring, wherein the heterocyclyl moiety is a 5- or 6-membered comprising 1 or 2 heteroatoms selected from O and N, and wherein the heterocyclyl ring may be optionally substituted with a single R¹⁰ group.

In one set of embodiments, R⁹ is cyano, halogen, C₁-C₄alkyl, C₁-C₃alkoxy, C₁-C₃haloalkyl, C₁-C₂haloalkoxy, C₁-C₃alkoxyC₁-C₃alkyl, C₁-C₃alkylcarbonyl, or C₃-C₄cycloalkyl; or any two adjacent R⁹ groups together with the carbon atoms to which they are attached, may form a phenyl ring optionally substituted with a single R¹⁰ group. Preferably, R⁹ is cyano, halogen, C₁-C₃alkyl, C₁-C₃alkoxy, C₁-C₂haloalkyl, C₁-C₂haloalkoxy, C₁-C₂alkoxyC₁-C₂alkyl, C₁-C₂alkylcarbonyl, or C₃-C₄cycloalkyl; or any two adjacent R⁹ groups together with the carbon atoms to which they are attached, may form a phenyl ring optionally substituted with a single R¹⁰ group.

R¹⁰ is halogen, C₁-C₃alkyl, or C₁-C₃alkoxy. Preferably, R¹⁰ is halogen or C₁-C₃alkoxy. More preferably, R¹⁰ is halogen or methoxy. Even more preferably, R¹⁰ is fluoro or methoxy.

In a compound of formula (I) according to the present invention, preferably:
R¹ is ethyl;
R² is 3,4-dichlorophenyl or 3,4-difluorophenyl;
R⁴ is heteroaryl wherein the heteroaryl moiety is a 5-membered aromatic monocyclic ring comprising 2 or 3 nitrogen atoms, and wherein the heteroaryl moieties are attached to the rest of the molecule through a nitrogen atom in the heteroaryl ring, and wherein the heteroaryl moieties may each be optionally substituted with 1, 2, or 3 groups, which may be the same or different, represented by R⁹;
R⁵ is methyl;
R⁹ is formyl, cyano, nitro, hydroxy, halogen, C₁-C₆alkyl, C₁-C₆alkoxy, C₁-C₆haloalkyl, C₁-C₆haloalkoxy, C₂-C₆alkenyloxy, cyanoC₁-C₄alkoxy, C₁-C₄alkoxyC₁-C₄alkyl, C₁-C₆alkoxyC₁-C₆alkoxy, C₁-C₄alkoxycarbonylC₁-C₄alkoxy, C₁-C₆alkoxycarbonyl, C₁-C₄alkylsulfanyl, C₁-C₄alkylsulfinyl, C₁-C₄alkylsulfonyl, C₁-C₄alkylsulfonamido, C₁-C₆alkylcarbonyl, C₁-C₆alkylaminocarbonyl, C₁-C₆alkylcarbonylamino, C₁-C₆alkylamino, C₁-C₆alkoxyC₁-C₆alkylaminocarbonyl, C₁-C₄alkoxyC₁-C₄alkylcarbonylamino, C₂-C₆alkenylcarbonylamino, C₃-C₆cycloalkyl, C₃-C₆cycloalkylaminocarbonyl, N,N-di(C₁-C₃alkyl)amino, N,N-di(C₁-C₃alkyl)aminocarbonyl, or benzyloxy; or
any two adjacent R⁹ groups together with the carbon atoms to which they are attached, may form a C₃-C₆cycloalkyl ring or phenyl ring, wherein the C₃-C₆cycloalkyl and phenyl moieties may be optionally substituted with 1, 2, or 3 groups, which may be the same or different, represented by R¹⁰; or
any two adjacent R⁹ groups together with the carbon atoms to which they are attached, may form a 5- or 6-membered heteroaryl ring, wherein the heteroaryl moiety is a 5- or 6-membered aromatic ring which comprises 1, 2, or 3 heteroatoms individually selected from N, O and S, and wherein the heteroaryl moiety may be optionally substituted with 1, 2, or 3 groups, which may be the same or different, represented by R¹⁰; or
any two adjacent R⁹ groups together with the carbon atoms to which they are attached, may form a 5- or 6-membered heterocyclyl ring, wherein the heterocyclyl moiety is a 5- or 6-membered comprising 1 or 2 heteroatoms selected from O and N, and wherein the heterocyclyl ring may be optionally substituted with 1, 2, or 3 groups, which may be the same or different, represented by R¹⁰;
R¹⁰ is halogen or C₁-C₃alkoxy.

In another set of embodiments,
R¹ is ethyl;
R² is 3,4-dichlorophenyl;
R⁴ is 3-(trifluoromethyl)pyrazol-1-yl, 3-chloropyrazol-1-yl, or 4-chloropyrazol-1-yl; and
R⁵ is methyl.

Compounds of the invention can be made as shown in the following schemes, in which, unless otherwise stated, the definition of each variable is as defined above for a compound of Formula (I). General methods for the production of compounds of Formula (I) are described below. Unless otherwise stated in the text, R¹, R², R⁴, and R⁵, are as defined hereinbefore. The starting materials used for the preparation of the compounds of the invention may be purchased from usual commercial suppliers or may be prepared by known methods. The starting materials as well as the intermediates may be purified before use in the next step by state of the art methodologies such as chromatography, crystallisation, distillation and filtration.

Compounds of Formula (I) according to the present invention may be prepared from compounds of Formula (A) as shown below in Scheme 1.

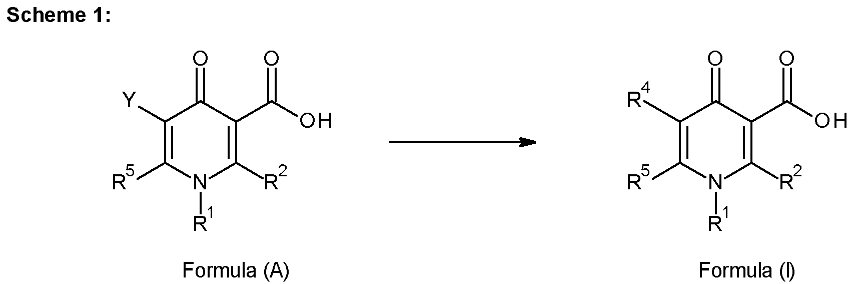

Compounds of Formula (I) wherein R⁴ is defined as above may be prepared from compounds of Formula (A) wherein Y is I (iodo) *via* an Ullmann type cross-coupling reaction. Typically the reaction is performed by reaction of a compound of Formula (A) with a cyclic NH-heterocycle (such as a pyrazole) in the presence of a copper salt (such as copper (I) iodide) and a ligand (such as *N,N'*-bis(2,6-dimethoxyphenyl)oxamide) in the presence of a base (such as potassium carbonate) in a suitable organic solvent (such as dimethyl sulfoxide) at elevated temperature. This is shown in Scheme 1 above.

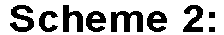

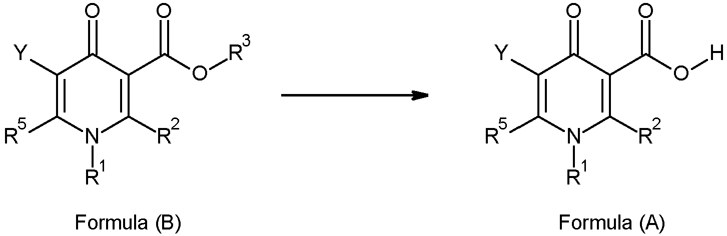

Compounds of Formula (A) wherein Y is I (iodo), may be prepared by hydrolysis of a compound of Formula (B) wherein R³ is C₁-C₆alkyl, with a suitable base (such as sodium hydroxide or lithium hydroxide) or with a suitable acid (such as trifluoroacetic acid, hydrochloric acid, formic acid or sulfuric acid) in a suitable solvent (such as methanol, ethanol, dichloromethane, chloroform, ethyl acetate or tetrahydrofuran) with an optional co-solvent (such as water). This is shown in Scheme 2 above.

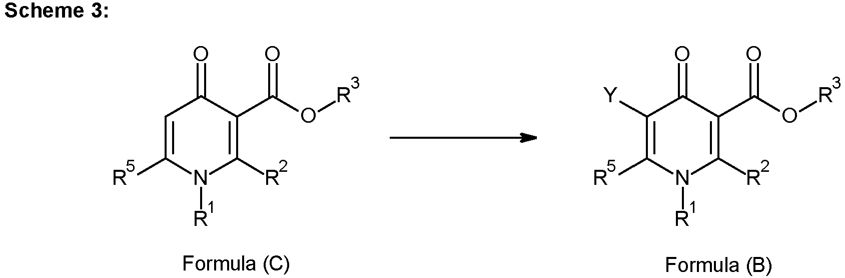

Compounds of Formula (B) wherein Y is I (iodo) may be prepared by treatment of compounds of Formula (C) with a suitable halogenating agent (such as N-iodo succinimide) in a suitable solvent (such as acetonitrile or trifluoroacetic acid). This is shown in Scheme 3 above.

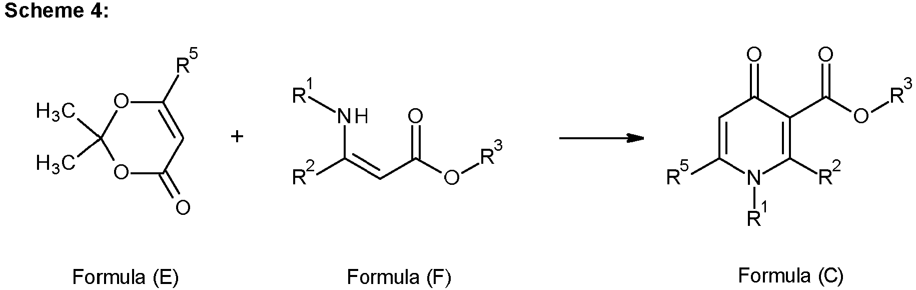

Compounds of Formula (C) may be prepared by reacting a compound of Formula (E) with a compound of Formula (F) without a solvent at an elevated temperature (for example 120 °C). Compounds of formula E are commercially available or may be prepared by methods familiar to persons skilled in the art. This is shown in Scheme 4 above.

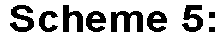

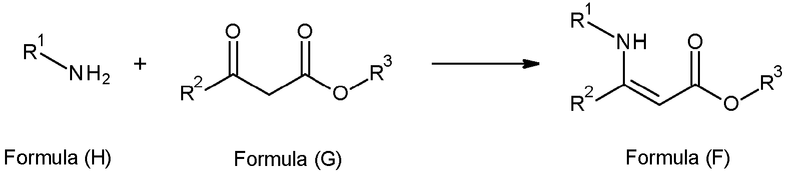

Compounds of Formula (F) maybe be prepared from reaction of β-keto esters of Formula (G) with an amine salt. The amine salts can be prepared *in situ* by acidification of amines of Formula (H) with a suitable acid (such as acetic acid). These amine salts may then be reacted with compounds of Formula (G) in a suitable solvent (such as toluene) in the presence of an acid (such as acetic acid) and a drying agent (such as 4Å molecular sieves. Compounds of Formula (G) are commercially available or may be prepared using conditions described below. Compounds of Formula (H) are commercially available or may be prepared by methods familiar to persons skilled in the art. This is shown in Scheme 5 above.

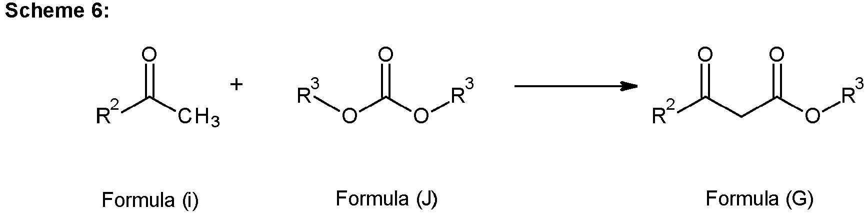

Compounds of Formula (G) may be prepared by treatment of ketones of Formula (i) with a base (such as sodium hydride) in the presence of dialkyl carbonates of Formula (J) (such as dimethyl carbonate). Compounds of Formula (i) and Formula (J) are commercially available or may be prepared by methods familiar to persons skilled in the art. This is shown in Scheme 6 above.

The present invention still further provides a method of controlling weeds at a locus said method comprising application to the locus of a weed controlling amount of a composition comprising a compound of Formula (I). Moreover, the present invention may further provide a method of selectively controlling weeds at a locus comprising useful (crop) plants and weeds, wherein the method comprises application to the locus of a weed controlling amount of a composition according to the present invention. 'Controlling' means killing, reducing or retarding growth or preventing or reducing germination. It is noted that the compounds of the present invention show a much improved selectivity compared to known, structurally similar compounds. Generally the plants to be controlled are unwanted plants (weeds). 'Locus' means the area in which the plants are growing or will grow. The application may be applied to the locus pre-emergence and/or postemergence of the crop plant. Some crop plants may be inherently tolerant to herbicidal effects of compounds of Formula (I).

The rates of application of compounds of Formula (I) may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2500 g/ha, especially from 25 to 1000 g/ha, more especially from 25 to 250 g/ha.

The application is generally made by spraying the composition, typically by tractor mounted sprayer for large areas, but other methods such as dusting (for powders), drip or drench can also be used.

The term "useful plants" is to be understood as also including useful plants that have been rendered tolerant to herbicides like bromoxynil or classes of herbicides such as, for example, 4-Hydroxyphenylpyruvate dioxygenase (HPPD) inhibitors, ALS inhibitors, for example primisulfuron, prosulfuron and trifloxysulfuron, 5-enol-pyrovyl-shikimate-3-phosphate-synthase (EPSPS) inhibitors, glutamine synthetase (GS) inhibitors or protoporphyrinogen-oxidase (PPO) inhibitors as a result of conventional methods of breeding or genetic engineering. An example of a crop that has been rendered tolerant to imidazolinones, e.g. imazamox, by conventional methods of breeding (mutagenesis) is Clearfield^{®} summer rape (Canola). Examples of crops that have been rendered tolerant to herbicides or classes of herbicides by genetic engineering methods include glyphosate- and glufosinate-resistant maize varieties commercially available under the trade names RoundupReady^{®}, Herculex I^{®} and LibertyLink^{®}.

The term "useful plants" is to be understood as also including useful plants which have been so transformed by the use of recombinant DNA techniques that they are capable of synthesising one or more selectively acting toxins, such as are known, for example, from toxin-producing bacteria, especially those of the genus Bacillus.

Examples of such plants are: YieldGard^{®} (maize variety that expresses a CryIA(b) toxin); YieldGard Rootworm^{®} (maize variety that expresses a CryllIB(b1) toxin); YieldGard Plus^{®} (maize variety that expresses a CryIA(b) and a CryllIB(b1) toxin); Starlink^{®} (maize variety that expresses a Cry9(c) toxin); Herculex I^{®} (maize variety that expresses a CrylF(a2) toxin and the enzyme phosphinothricine N-acetyltransferase (PAT) to achieve tolerance to the herbicide glufosinate ammonium); NuCOTN 33B^{®} (cotton variety that expresses a CryIA(c) toxin); Bollgard I^{®} (cotton variety that expresses a CryIA(c) toxin); Bollgard II^{®} (cotton variety that expresses a CryIA(c) and a CryllA(b) toxin); VIPCOT^{®} (cotton variety that expresses a VIP toxin); NewLeaf^{®} (potato variety that expresses a CrylllA toxin); NatureGard^{®} Agrisure^{®} GT Advantage (GA21 glyphosate-tolerant trait), Agrisure^{®} CB Advantage (Bt11 corn borer (CB) trait), Agrisure^{®} RW (corn rootworm trait) and Protecta^{®}.

Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

The compounds of Formula (I) (or compositions comprising such) can be used to control unwanted plants (collectively, 'weeds'). The weeds to be controlled may be both monocotyledonous species, for example *Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum,* and dicotyledonous species, for example *Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium.*

Compounds of Formula (I) may be used in unmodified form or, preferably, together with the adjuvants conventionally employed in the art of formulation to provide herbicidal compositions, using formulation adjuvants, such as carriers, solvents and surface-active agents (SAA). The invention therefore further provides a herbicidal composition, comprising at least one compound Formula (I) and an agriculturally acceptable carrier and optionally an adjuvant. An agricultural acceptable carrier is for example a carrier that is suitable for agricultural use. Agricultural carriers are well known in the art.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types. These include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a soluble powder (SP), a wettable powder (WP) and a soluble granule (SG). The formulation type chosen in any instance will depend upon the particular purpose envisaged and the physical, chemical and biological properties of the compound of Formula (I).

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from pre-formed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SAAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SAAs, to produce spontaneously a thermodynamically stable isotropic liquid formulation. A compound of Formula (I) is present initially in either the water or the solvent/SAA blend. Suitable solvents for use in MEs include those hereinbefore described for use in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SAAs), spray additives based on oils, for example certain mineral oils or natural plant oils (such as soy bean and rape seed oil), modified plant oils such as methylated rape seed oil (MRSO), and blends of these with other bio-enhancing adjuvants (ingredients which may aid or modify the action of a compound of Formula (I).

Wetting agents, dispersing agents and emulsifying agents may be SAAs of the cationic, anionic, amphoteric or non-ionic type.

Suitable SAAs of the cationic type include quaternary ammonium compounds (for example cetyltrimethyl ammonium bromide), imidazolines and amine salts.

Suitable anionic SAAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-isopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately diesters), for example the reaction between lauryl alcohol and tetraphosphoric acid; additionally these products may be ethoxylated), sulphosuccinamates, paraffin or olefine sulphonates, taurates, lignosulphonates and phosphates / sulphates of tristyrylphenols.

Suitable SAAs of the amphoteric type include betaines, propionates and glycinates. Suitable SAAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); lecithins and sorbitans and esters thereof, alkyl polyglycosides and tristyrylphenols. Suitable suspending agents include hydrophilic colloids (such as polysaccharides, polyvinylpyrrolidone or sodium carboxymethylcellulose) and swelling clays (such as bentonite or attapulgite).

The compounds of the present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, benquitrione, bensulfuron (including bensulfuron-methyl), bentazone, bicyclopyrone, bilanafos, bipyrazone, bispyribac-sodium, bixlozone, broclozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, dioxopyritrione, diquat dibromide, diuron, epyrifenacil, ethalfluralin, ethofumesate, fenoxaprop (including fenoxaprop-P-ethyl), fenoxasulfone, fenpyrazone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), fluchloraminopyr (including fluchloramino-tefuryl), flufenacet, flufenoximacil, flumetsulam, flumioxazin, fluometuron, fomesafen flupyrsulfuron (including flupyrsulfuron-methyl-sodium), fluroxypyr (including fluroxypyr-meptyl), flusulfinam, fomesafen, foramsulfuron, glufosinate (including L-glufosinate and the ammonium salts of both), glyphosate (including the diammonium, isopropylammonium and potassium salts thereof), halauxifen (including halauxifen-methyl), haloxyfop (including haloxyfop-methyl), hexazinone, hydantocidin, icafolin (including icafolin-methyl), imazamox (including R-imazamox), imazapic, imazapyr, imazethapyr, indaziflam, indolauxipyr (including indolauxipyr-cyanomethyl), iodosulfuron (including iodosulfuron-methyl-sodium), iofensulfuron (including iofensulfuron-sodium), ioxynil, iptriazopyrid, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyraquinate, pyrasulfotole, pyridate, pyriftalid, pyriflubenzoxim, pyrimisulfan, pyroxasulfone, pyroxsulam, quinclorac, quinmerac, quizalofop (including quizalofop-P-ethyl and quizalofop-P-tefuryl), rimisoxafen, rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, tripyrasulfone, 3-(2-chloro-4-fluoro-5-(3-methyl-2,6-dioxo-4-trifluoromethyl-3,6-dihydropyrimidin-1(2H)-yl)phenyl)-5-methyl-4,5-dihydroisoxazole-5-carboxylic acid ethyl ester, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, (1RS,5SR)-3-[2-methoxy-4-(prop-1-yn-1-yl)phenyl]-4-oxobicyclo[3.2.1]oct-2-en-2-yl methyl carbonate, ethyl-2-[[3-[[3-chloro-5-fluoro-6-[3-methyl-2,6-dioxo-4-(trifluoromethyl)pyrimidin-1-yl]-2-pyridyl]oxy]acetate, methyl 2-[2-[2-bromo-4-fluoro-5-[3-methyl-2,6-dioxo-4-(trifluoromethyl)pyrimidin-1-yl]phenoxy]phenoxy]-2-methoxy-acetate, 6-chloro-4-(2,7-dimethyl-1-naphthyl)-5-hydroxy-2-methyl-pyridazin-3-one, (2-fluorophenyl)methyl 6-amino-5-chloro-2-(4-chloro-2-fluoro-3-methoxy-phenyl)pyrimidine-4-carboxylate, 6-amino-5-chloro-2-(4-chloro-2-fluoro-3-methoxyphenyl)pyrimidine-4-carboxylic acid, and methyl 3-[2-chloro-5-[3,6-dihydro-3-methyl-2,6-dioxo-4-(trifluoromethyl)-1(2H)-pyrimidinyl]-4-fluorophenyl]-3a,4,5,6-tetrahydro-6-methyl-6aH-cyclopent[d]isoxazole-6a-carboxylate.

The mixing partners of the compound of Formula (I) may also be in the form of esters or salts, as mentioned e.g. in The Pesticide Manual, Nineteenth Edition, British Crop Protection Council, 2021. The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula (I) with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil.

Particularly preferred are mixtures of a compound of Formula (I) with cyprosulfamide, isoxadifen-ethyl, cloquintocet-mexyl and/or metcamifen.

The safeners of the compound of Formula (I) may also be in the form of esters or salts, as mentioned e.g. in The Pesticide Manual, 19th Edition (BCPC), 2021. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048.

Preferably the mixing ratio of compound of Formula (I) to safener is from 100:1 to 1:10, especially from 20:1 to 1:1.

The compounds of Formula (I) are normally used in the form of agrochemical compositions and can be applied to the crop area or plant to be treated, simultaneously or in succession with further compounds. These further compounds can be e.g. fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The term "locus" as used herein means fields in or on which plants are growing, or where seeds of cultivated plants are sown, or where seed will be placed into the soil. It includes soil, seeds, and seedlings, as well as established vegetation.

The term "plants" refers to all physical parts of a plant, including seeds, seedlings, saplings, roots, tubers, stems, stalks, foliage, and fruits.

The term "plant propagation material" is understood to denote generative parts of the plant, such as seeds, which can be used for the multiplication of the latter, and vegetative material, such as cuttings or tubers, for example potatoes. There may be mentioned for example seeds (in the strict sense), roots, fruits, tubers, bulbs, rhizomes and parts of plants. Germinated plants and young plants which are to be transplanted after germination or after emergence from the soil, may also be mentioned. These young plants may be protected before transplantation by a total or partial treatment by immersion. Preferably "plant propagation material" is understood to denote seeds.

Pesticidal agents referred to herein using their common name are known, for example, from "The Pesticide Manual", 15th Ed., British Crop Protection Council 2009.

The compounds of formula (I) may be used in unmodified form or, preferably, together with the adjuvants conventionally employed in the art of formulation. To this end, they may be conveniently formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants, e.g., for agricultural use, can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of Formula (I) are normally used in the form of compositions and can be applied to the crop area or plant to be treated, simultaneously or in succession with further compounds. These further compounds can be, e.g., fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The compound of Formula (I) may be the sole active ingredient of a composition or it may be admixed with one or more additional active ingredients such as a pesticide, fungicide, synergist, herbicide or plant growth regulator where appropriate. An additional active ingredient may, in some cases, result in unexpected synergistic activities.

In general, the formulations include from 0.01 to 90% by weight of active agent, from 0 to 20% agriculturally acceptable surfactant and 10 to 99.99% solid or liquid formulation inerts and adjuvant(s), the active agent consisting of at least the compound of formula (I) together with component (B) and (C), and optionally other active agents, particularly microbiocides or conservatives or the like. Concentrated forms of compositions generally contain in between about 2 and 80%, preferably between about 5 and 70% by weight of active agent. Application forms of formulation may for example contain from 0.01 to 20% by weight, preferably from 0.01 to 5% by weight of active agent. Whereas commercial products will preferably be formulated as concentrates, the end user will normally employ diluted formulations.

The table below illustrates examples of individual compounds of Formula (I) according to the invention:

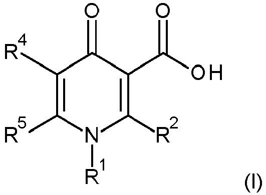

**Table 1: Individual compounds of Formula (I) according to the invention**

| **Cpd No.** | **R¹** | **R²** | **R⁴** | **R⁵** |
|---|---|---|---|---|
| 001 | Et | (3-chloro-4-nitro-phenyl) | [3-(trifluoromethyl)pyrazol-1-yl] | Me |
| 002 | Et | (3-chloro-4-nitro-phenyl) | (3-chloropyrazol-1-yl) | Me |
| 003 | Et | (3,4-dichlorophenyl) | [3-(trifluoromethoxy)pyrazol-1-yl] | Me |
| 004 | Et | (3,4-dichlorophenyl) | [4-chloro-3-(trifluoromethoxy)pyrazol-1-yl] | Me |
| 005 | Et | (3,4-dichlorophenyl) | [5-chloro-3-(trifluoromethoxy)pyrazol-1-yl] | Me |
| 006 | Et | (3,4-dichlorophenyl) | [3-(trifluoromethyl)pyrazol-1-yl] | H |
| 007 | Et | (3,4-dichlorophenyl) | (3-chloropyrazol-1-yl) | H |
| 008 | Et | (3-chloro-4-nitro-phenyl) | [3-(trifluoromethyl)pyrazol-1-yl] | H |
| 009 | Et | (3,4-dichlorophenyl) | (3,5-dichloropyrazol-1-yl) | Me |
| 010 | Et | (3-chloro-4-nitro-phenyl) | (3,5-dichloropyrazol-1-yl) | Me |
| 011 | Me | (3,4-dichlorophenyl) | [3-(trifluoromethyl)pyrazol-1-yl] | Me |
| 012 | n-propyl | (3,4-dichlorophenyl) | [3-(trifluoromethyl)pyrazol-1-yl] | Me |
| 013 | allyl | (3,4-dichlorophenyl) | [3-(trifluoromethyl)pyrazol-1-yl] | Me |
| 014 | 2-methoxyethyl | (3,4-dichlorophenyl) | [3-(trifluoromethyl)pyrazol-1-yl] | Me |
| 015 | methoxy | (3,4-dichlorophenyl) | [3-(trifluoromethyl)pyrazol-1-yl] | Me |
| 016 | prop-2-ynyl | (3,4-dichlorophenyl) | [3-(trifluoromethyl)pyrazol-1-yl] | Me |
| 017 | Et | (3,4-dichlorophenyl) | [3-(difluoromethoxy)pyrazol-1-yl] | Me |
| 018 | Et | (3,4-dichlorophenyl) | [3-(trifluoromethoxy)pyrazol-1-yl] | Me |
| 019 | Et | (3,4-dichlorophenyl) | [4-(difluoromethoxy)pyrazol-1-yl] | Me |
| 020 | Et | (3,4-dichlorophenyl) | [4-(trifluoromethoxy)pyrazol-1-yl] | Me |
| 021 | Et | (3,4-difluorophenyl) | [4-(difluoromethoxy)pyrazol-1-yl] | Me |
| 022 | Et | (3,4-difluorophenyl) | [4-(trifluoromethoxy)pyrazol-1-yl] | Me |
| 023 | Et | (3,4-dichlorophenyl) | (3-methoxy-1,2,4-triazol-1-yl) | Me |
| 024 | Et | (3,4-dichlorophenyl) | [3-(trifluoromethoxy)-1,2,4-triazol-1-yl] | Me |
| 025 | Et | (3,4-dichlorophenyl) | [3-(trifluoromethyl)pyrazol-1-yl] | H |
| 026 | Et | (3,4-dichlorophenyl) | (3-chloropyrazol-1-yl) | H |

Table A-1 provides 26 compounds A-1.001 to A.1.026 of Formula (I) wherein R¹, R², R⁴, and R⁵ are as defined in Table 1.

### Formulation Examples

| Wettable powders | a) | b) | c) |
|---|---|---|---|
| active ingredient [compound of formula (I)] | 25 % | 50 % | 75 % |
| sodium lignosulfonate | 5 % | 5 % | - |
| sodium lauryl sulfate | 3 % | - | 5 % |
| sodium diisobutylnaphthalenesulfonate | - | 6 % | 10 % |
| phenol polyethylene glycol ether | - | 2 % | - |
| (7-8 mol of ethylene oxide) | | | |
| highly dispersed silicic acid | 5 % | 10 % | 10 % |
| Kaolin | 62 % | 27 % | - |

The active ingredient is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredient [compound of formula (I)] | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | | 20 % |

The active ingredient is thoroughly mixed with the adjuvants and the mixture is thoroughly ground in a suitable mill, affording powders that can be used directly for seed treatment.

### Emulsifiable concentrate

| | |
|---|---|
| active ingredient [compound of formula (I)] | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredient [compound of formula (I)] | 5 % | 6 % | 4 % |
| talcum | 95 % | - | - |
| Kaolin | - | 94 % | - |
| mineral filler | - | - | 96 % |

Ready-for-use dusts are obtained by mixing the active ingredient with the carrier and grinding the mixture in a suitable mill. Such powders can also be used for dry dressings for seed.

### Extruder granules

| | |
|---|---|
| Active ingredient [compound of formula (I)] | 15 % |
| sodium lignosulfonate | 2 % |
| carboxymethylcellulose | 1 % |
| Kaolin | 82 % |

The active ingredient is mixed and ground with the adjuvants, and the mixture is moistened with water. The mixture is extruded and then dried in a stream of air.

### Coated granules

| | |
|---|---|
| Active ingredient [compound of formula (I)] | 8 % |
| polyethylene glycol (mol. wt. 200) | 3 % |
| Kaolin | 89 % |

The finely ground active ingredient is uniformly applied, in a mixer, to the kaolin moistened with polyethylene glycol. Non-dusty coated granules are obtained in this manner.

### Suspension concentrate

| | |
|---|---|
| active ingredient [compound of formula (I)] | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredient [compound of formula (I)] | 40 % |
| propylene glycol | 5 % |
| copolymer butanol PO/EO | 2 % |
| tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of a combination of the compound of formula (I) are mixed with 2 parts of an aromatic solvent and 7 parts of toluene diisocyanate/polymethylene-polyphenylisocyanate-mixture (8:1). This mixture is emulsified in a mixture of 1.2 parts of polyvinyl alcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

### Examples

The following non-limiting examples provide specific synthesis methods for representative compounds of the present invention, as referred to in Table 2 below.

### List of Abbreviations

Å = Angstrom, brs = broad singlet, °C = degrees Celsius, d = doublet, dd = doublet of doublets, DMSO = dimethyl sulfoxide, HPLC = high performance liquid chromatography, M = molar, m = multiplet, MHz = megahertz, mL = milliliter, q = quartet, s = singlet, t = triplet, THF = tetrahydrofuran.

### Example 1: Synthesis of 2-(3,4-dichlorophenyl)-1-ethyl-6-methyl-4-oxo-5-[3-(trifluoromethyl)pyrazol-1-yl]pyridine-3-carboxylic acid (Compound 1)

### Step 1: Synthesis of methyl 3-(3,4-dichlorophenyl)-3-oxo-propanoate

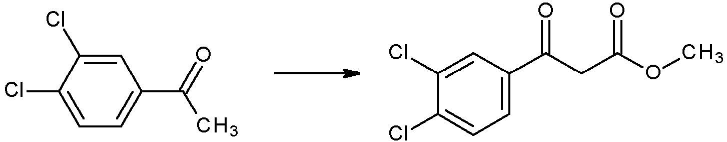

To a stirred solution of 1-(3,4-dichlorophenyl)ethanone (5.00 g, 26.5 mmol) and dimethyl carbonate (40 mL, 466 mmol) under nitrogen and cooled to 0°C was added portion-wise sodium hydride (3.17 g, 79.5 mmol, 60 mass%). The reaction mixture was allowed to warm to room temperature and stirred for 16 hours. Overnight the reaction mixture became a solid paste which was not possible to stir. More dimethyl carbonate (10 mL) was added in an attempt to create a mobile slurry for quenching. The reaction mixture was cooled to 0°C and quenched by addition of water (25 mL) under nitrogen. The reaction mixture was acidified to pH3 by addition of aqueous hydrochloric acid (2M) and then extracted with ethyl acetate. The organic extract was dried over magnesium sulfate and evaporated to dryness under reduced pressure. The crude residue was purified by flash chromatography on silica gel using a gradient of 0-15% ethyl acetate in isohexane as eluent to give methyl 3-(3,4-dichlorophenyl)-3-oxo-propanoate (mixture of tautomers) as a colourless liquid.

Enol: ¹H NMR (400 MHz, chloroform) δ = 12.47 (s, 1H), 7.87 (d, 1H), 7.59 (m, 3H), 7.49 (d, 1H), 5.65 (s, 1H), 3.82 (s, 3H)

Keto: ¹H NMR (400 MHz, chloroform) δ = 8.03 (d, 1H), 7.77 (m, 1H), 7.58 (d, 2H), 3.97 (s, 2H), 3.76 (s, 3H).

### Step 2: Synthesis of methyl (Z)-3-(3,4-dichlorophenyl)-3-(ethylamino)prop-2-enoate

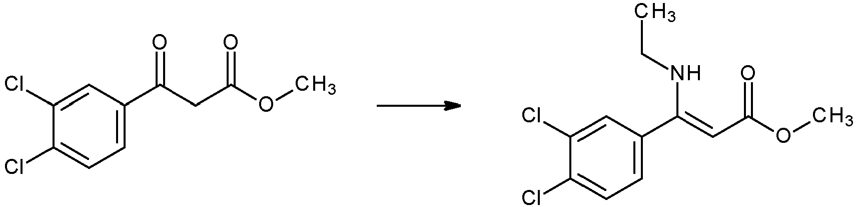

To a stirred solution of ethylamine (2M in THF) (12.2 mL, 24.34 mmol) at 0°C was added dropwise acetic acid (1.39 mL, 24.3 mmol). The mixture was allowed to warm to room temperature and stirred for 1 hour before being evaporated to dryness under reduced pressure to afford ethylammonium acetate (2.55 g, 24.3 mmol). The ethylammonium acetate (2.55 g, 24.3 mmol) was added to a solution of methyl 3-(3,4-dichlorophenyl)-3-oxo-propanoate (2.00 g, 8.09 mmol) in toluene (20 mL) followed by addition of acetic acid (0.46 mL, 8.09 mmol) and powdered 4Å molecular sieves. The reaction mixture was heated at reflux for 18 hours. The cooled reaction mixture was diluted with ethyl acetate, filtered and washed with saturated aqueous sodium bicarbonate solution. The phases were separated, and the aqueous phase was extracted with ethyl acetate (x3). The combined organic extracts were washed with brine, dried over magnesium sulfate and evaporated to dryness under reduced pressure. The crude residue was purified by flash chromatography on silica gel using a gradient of 0-10% ethyl acetate in isohexane as eluent to give methyl (Z)-3-(3,4-dichlorophenyl)-3-(ethylamino)prop-2-enoate as a pale yellow oil. ¹H NMR (400 MHz, chloroform) δ = 8.37 (br s, 1H), 7.48 (d, 1H), 7.46 (d, 1H), 7.20 (m, 1H), 4.55 (s, 1H), 3.68 (s, 3H), 3.07 (m, 2H), 1.13 - 1.09 (m, 3H).

### Step 3: Synthesis of methyl 2-(3,4-dichlorophenyl)-1-ethyl-6-methyl-4-oxo-pyridine-3-carboxylate

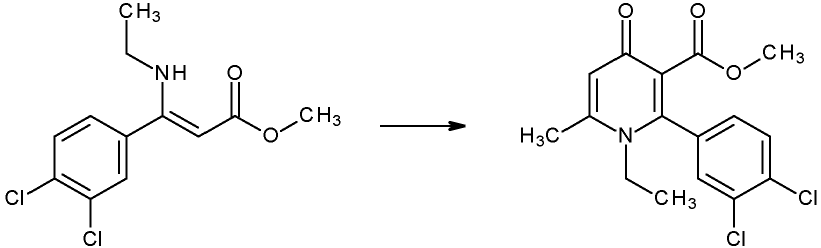

A stirred mixture of methyl (Z)-3-(3,4-dichlorophenyl)-3-(ethylamino)prop-2-enoate (1.50 g, 5.5 mmol) and 2,2,6-trimethyl-1,3-dioxin-4-one (0.82 g, 5.5 mmol) under nitrogen were heated at 120 °C for 3 hours. The cooled reaction mixture was evaporated to dryness under reduced pressure. The crude residue was purified by flash chromatography on silica gel using a gradient of 0-10% methanol in dichloromethane as eluent to give methyl 2-(3,4-dichlorophenyl)-1-ethyl-6-methyl-4-oxo-pyridine-3-carboxylate as an off-white solid. ¹H NMR (400 MHz, chloroform) δ = 7.56 (d, 1H), 7.50 (d, 1H), 7.24 (m, 1H), 6.41 (s, 1H), 3.72 (q, 2H), 3.55 (s, 3H), 2.42 (s, 3H), 1.13 (t, 3H).

### Step 4: Synthesis of methyl 2-(3,4-dichlorophenyl)-1-ethyl-5-iodo-6-methyl-4-oxo-pyridine-3-carboxylate

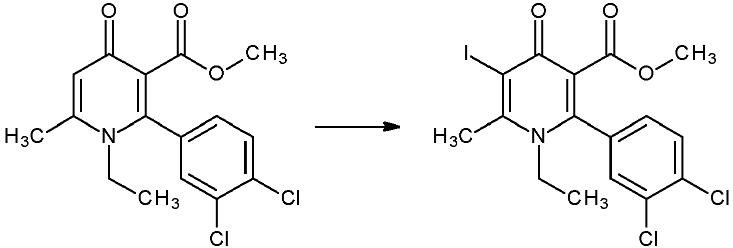

To a solution of methyl 2-(3,4-dichlorophenyl)-1-ethyl-6-methyl-4-oxo-pyridine-3-carboxylate (5.60 g, 16.5 mmol) in acetonitrile (56.0 mL) at room temperature and under nitrogen was added 1-iodopyrrolidine-2,5-dione (3.70 g, 16.5 mmol) followed by 2,2,2-trifluoroacetic acid (0.564 g, 0.381 mL, 4.94 mmol). The reaction mixture was heated at 80 °C for 36 hours and then stirred at room temperature for 48 hours. The cooled reaction mixture was quenched by addition of saturated aqueous sodium hydrogen carbonate solution (200 mL) and extracted with dichloromethane (x3). The combined organic extracts were washed with saturated sodium thiosulfate solution then brine, dried over magnesium sulfate, filtered and evaporated under reduced pressure. The crude residue was purified by flash chromatography on silica gel using a gradient of 0-100% ethyl acetate in cyclohexane as eluent to give methyl 2-(3,4-dichlorophenyl)-1-ethyl-5-iodo-6-methyl-4-oxo-pyridine-3-carboxylate as a white solid. ¹H NMR (400 MHz, chloroform) δ = 7.57 (d, 1H), 7.49 (d, 1H), 7.23 (m, 1H), 3.89 (q, 2H), 3.57 (s, 3H), 2.88 (s, 3H), 1.17 (t, 3H).

### Step 5: Synthesis of 2-(3,4-dichlorophenyl)-1-ethyl-5-iodo-6-methyl-4-oxo-pyridine-3-carboxylic acid

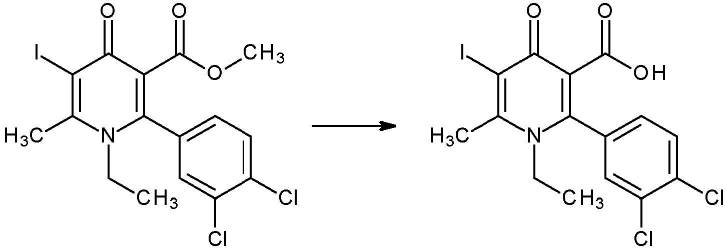

To a solution of methyl 2-(3,4-dichlorophenyl)-1-ethyl-5-iodo-6-methyl-4-oxo-pyridine-3-carboxylate (12.60 g, 27.0 mmol) in methanol (113 mL) was added a solution of lithium hydroxide (2.59 g, 108 mmol) in water (31.5 mL). The resultant white suspension was heated at 80 °C for 2 hours and then stood at room temperature for 18 hours. The cooled reaction mixture was acidified to pH 1 by addition of aqueous hydrogen chloride solution (2M). The resultant white precipitate was collected by filtration, washed with water and air-dried to afford 2-(3,4-dichlorophenyl)-1-ethyl-5-iodo-6-methyl-4-oxo-pyridine-3-carboxylic acid as a white solid. ¹H NMR (500 MHz, chloroform) δ = 16.06 (brs, 1H), 7.60 (d, 1H), 7.34 (d, 1H), 7.10 (dd, 1H), 4.04 - 3.99 (m, 2H), 2.99 (s, 3H), 1.23 (t, 3H)

### Step 6: Synthesis of 2-(3,4-dichlorophenyl)-1-ethyl-6-methyl-4-oxo-5-[3-(trifluoromethyl)pyrazol-1-yl]pyridine-3-carboxylic acid

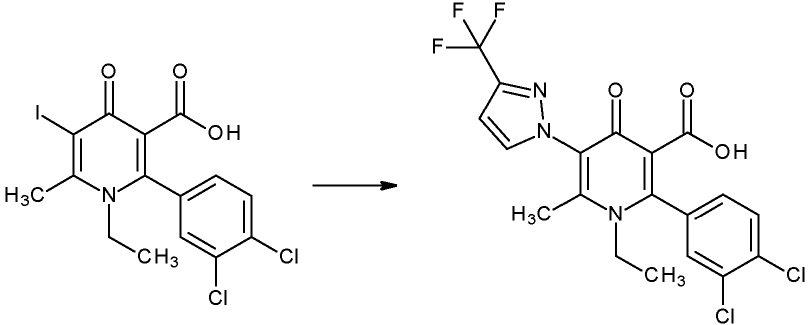

A mixture of 2-(3,4-dichlorophenyl)-1-ethyl-5-iodo-6-methyl-4-oxo-pyridine-3-carboxylic acid (0.25 g, 0.553 mmol), 3-(trifluoromethyl)-1H-pyrazole (0.27 g, 1.99 mmol), copper(I) iodide (0.106 g, 0.553 mmol), *N,N'*-bis(2,6-dimethoxyphenyl)oxamide (0.399 g, 1.11 mmol) and potassium carbonate (0.232 g, 1.66 mmol) was purged with nitrogen (x3) before addition of dimethyl sulfoxide (3.75 mL). The reaction mixture was heated with stirring at 120 °C for 18 hours. The cooled reaction mixture was poured into water and diluted with diethyl ether. The aqueous phase was acidified to pH 1 by addition of aqueous hydrogen chloride solution (2M). The brown solid precipitate was removed by filtration and the organic phase was washed with brine, dried over magnesium sulfate, filtered and evaporated to dryness under reduced pressure. The crude residue was purified by mass-directed reverse phase HPLC to give 2-(3,4-dichlorophenyl)-1-ethyl-6-methyl-4-oxo-5-[3-(trifluoromethyl)pyrazol-1-yl]pyridine-3-carboxylic acid as a white solid. ¹H NMR (400 MHz, chloroform) δ = 7.85 - 7.80 (m, 1H), 7.62 (d, 1H), 7.38 (d, 1H), 7.13 (dd, 1H), 6.79 (d, 1H), 3.95 (q, 2H), 2.41 (s, 3H), 1.27 (t, 3H).

### Example 2: Synthesis of 5-(3-chloropyrazol-1-yl)-2-(3,4-dichlorophenyl)-1-ethyl-6-methyl-4-oxo-pyridine-3-carboxylic acid (Compound 2)

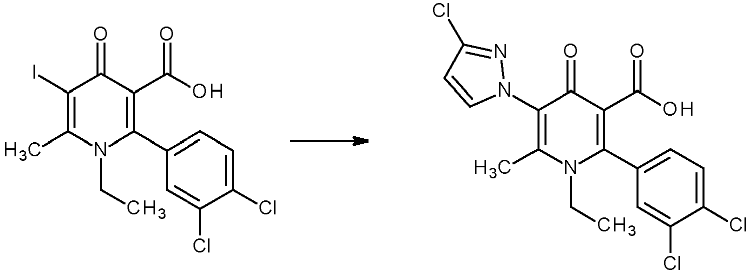

A mixture of 2-(3,4-dichlorophenyl)-1-ethyl-5-iodo-6-methyl-4-oxo-pyridine-3-carboxylic acid (0.250 g, 0.553 mmol), 3-chloro-1H-pyrazole (0.204 g, 1.99 mmol), copper(I) iodide (0.106 g, 0.553 mmol), *N,N'-*bis(2,6-dimethoxyphenyl)oxamide (0.399 g, 1.11 mmol) and potassium carbonate (0.232 g, 1.66 mmol) was purged with nitrogen (x3) before addition of DMSO (3.75 mL). The reaction mixture was heated at 120°C for 18 hours. The cooled reaction mixture was poured into water and diluted with diethyl ether. The aqueous phase was acidified to pH 1 by addition of aqueous hydrogen chloride solution (2M). The brown solid precipitate was removed by filtration and the organic phase was washed with brine, dried over magnesium sulfate, filtered and evaporated to dryness under reduced pressure. The crude residue was purified by mass-directed reverse phase HPLC to give 5-(3-chloropyrazol-1-yl)-2-(3,4-dichlorophenyl)-1-ethyl-6-methyl-4-oxo-pyridine-3-carboxylic acid as a white solid. ¹H NMR (400 MHz, chloroform) δ = 7.67 (d, 1H), 7.62 (d, 1H), 7.37 (d, 1H), 7.13 (dd, 1H), 6.47 (d, 1H), 3.95 (q, 2H), 2.47 (s, 3H), 1.26 (t, 3H).

### Example 3: Synthesis of 5-(3-chloropyrazol-1-yl)-2-(3,4-difluorophenyl)-1-ethyl-6-methyl-4-oxo-pyridine-3-carboxylic acid (Compound 4)

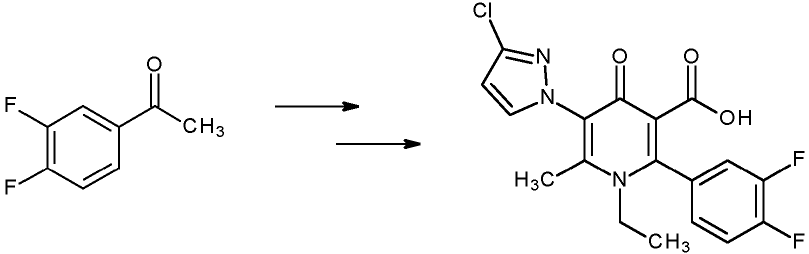

5-(3-chloropyrazol-1-yl)-2-(3,4-difluorophenyl)-1-ethyl-6-methyl-4-oxo-pyridine-3-carboxylic acid was prepared as for 5-(3-chloropyrazol-1-yl)-2-(3,4-dichlorophenyl)-1-ethyl-6-methyl-4-oxo-pyridine-3-carboxylic acid from 3',4'-difluoroacetophenone following the 6-step procedure herein described. ¹H NMR (400 MHz, chloroform) δ = 7.68 (d, 1H), 7.35 (td, 1H), 7.13 (ddd, 1H), 7.04 - 6.98 (m, 1H), 6.47 (d, 1H), 3.96 (q, 2H), 2.47 (s, 3H), 1.26 (t, 3H)

Throughout this description, temperatures are given in degrees Celsius (°C) and "m.p." means melting point.

**Table 2: ¹H NMR data for selected compounds according to the invention.**

| **Compound No.** | **Compound Name** | **Structure & ¹H NMR Data** |
|---|---|---|
| 1 | 2-(3,4-dichlorophenyl)-1-ethyl-6-methyl-4-oxo-5-[3-(trifluoromethyl)pyrazol-1-yl]pyridine-3-carboxylic acid | 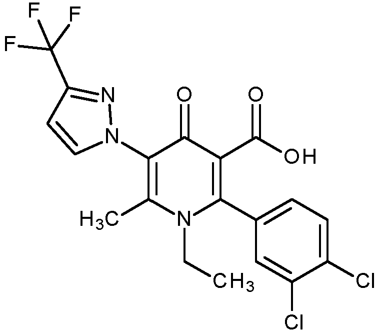 1H NMR (400 MHz, chloroform) δ = 7.85 - 7.80 (m, 1H), 7.62 (d, 1H), 7.38 (d, 1H), 7.13 (dd, 1H), 6.79 (d, 1H), 3.95 (q, 2H), 2.41 (s, 3H), 1.27 (t, 3H) |
| 2 | 5-(3-chloropyrazol-1-yl)-2-(3,4-dichlorophenyl)-1-ethyl-6-methyl-4-oxo-pyridine-3-carboxylic acid | 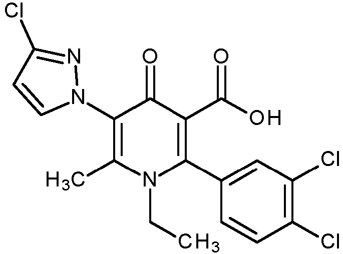 1H NMR (400 MHz, chloroform) δ = 7.67 (d, 1H), 7.62 (d, 1H), 7.37 (d, 1H), 7.13 (dd, 1H), 6.47 (d, 1H), 3.95 (q, 2H), 2.47 (s, 3H), 1.26 (t, 3H) |
| 3 | 5-(4-chloropyrazol-1-yl)-2-(3,4-dichlorophenyl)-1-ethyl-6-methyl-4-oxo-pyridine-3-carboxylic acid | 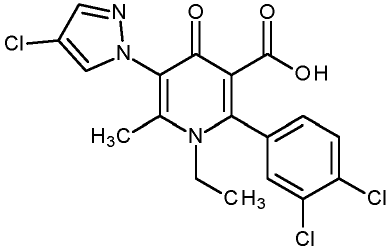 1H NMR (400 MHz, methanol) δ = 7.88 (s, 1H), 7.77 - 7.69 (m, 3H), 7.44 (dd, 1H), 3.99 (q, 2H), 2.32 (s, 3H), 1.22 (t, 3H) |
| 4 | 5-(3-chloropyrazol-1-yl)-2-(3,4-difluorophenyl)-1-ethyl-6-methyl-4-oxo-pyridine-3-carboxylic acid | 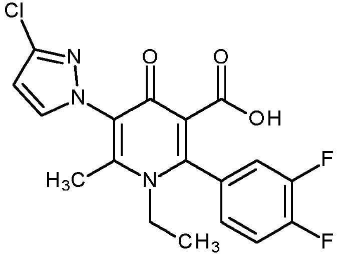 ¹H NMR (400 MHz, chloroform) δ = 7.68 (d, 1H), 7.35 (td, 1H), 7.13 (ddd, 1H), 7.04 - 6.98 (m, 1H), 6.47 (d, 1H), 3.96 (q, 2H), 2.47 (s, 3H), 1.26 (t, 3H) |
| 5 | 2-(3,4-dichlorophenyl)-1-ethyl-6-methyl-5-(4-methylpyrazol-1-yl)-4-oxo-pyridine-3-carboxylic acid | 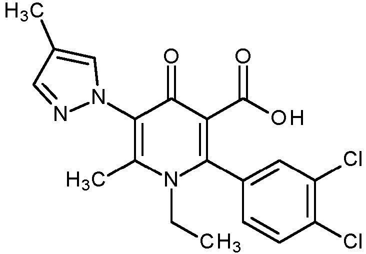 ¹H NMR (500 MHz, DMSO-d6) δ = 7.85 (d, 1H), 7.81 (d, 1H), 7.57 (d, 2H), 7.49 (dd, 1H), 3.88 (q, 2H), 2.24 (s, 3H), 2.12 (s, 3H), 1.11 (t, 3H) |
| 6 | 2-(3,4-dichlorophenyl)-1-ethyl-6-methyl-5-(3-methylpyrazol-1-yl)-4-oxo-pyridine-3-carboxylic acid | 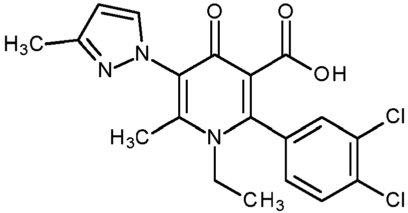 ¹H NMR (500 MHz, DMSO-d6) δ = 7.83 (d, 1H), 7.81 (d, 1H), 7.67 (d, 1H), 7.48 (dd,1H), 6.30 (d, 1H), 3.88 (q, 2H), 2.27 (s, 3H), 2.25 (s, 3H), 1.12 (t, 3H) |
| 7 | 2-(3,4-dichlorophenyl)-1-ethyl-5-(3-methoxypyrazol-1-yl)-6-methyl-4-oxo-pyridine-3-carboxylic acid | 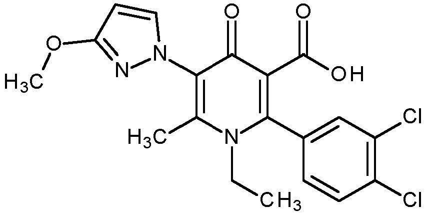 ¹H NMR (500 MHz, DMSO-d6) δ = 7.83 (d, 1H), 7.81 (d, 1H), 7.62 (d, 1H), 7.47 (dd, 1H), 5.96 (d, 1H), 3.88 (q, 2H), 3.81 (s, 3H), 2.32 (s, 3H), 1.12 (t, 3H) |
| 8 | 2-(3,4-dichlorophenyl)-1-ethyl-5-(3-isopropoxypyrazol-1-yl)-6-methyl-4-oxo-pyridine-3-carboxylic acid | 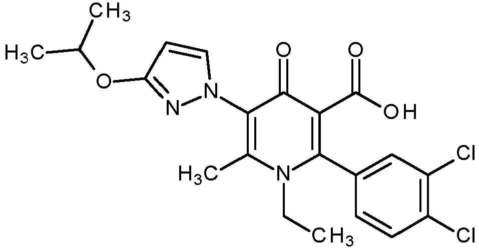 ¹H NMR (500 MHz, DMSO-d6) δ = 7.83 (d, 1H), 7.81 (d, 1H), 7.60 (d, 1H), 7.48 (dd, 1H), 5.91 (d, 1H), 4.73 - 4.68 (m, 1H), 3.87 (q, 2H), 2.32 (s, 3H), 1.30 (d, 6H), 1.16 - 1.07 (m, 3H) |
| 9 | 5-(3-cyclopropylpyrazol-1-yl)-2-(3,4-dichlorophenyl)-1-ethyl-6-methyl-4-oxo-pyridine-3-carboxylic acid | 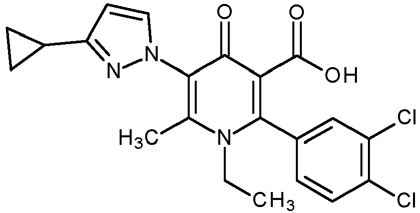 ¹H NMR (500 MHz, DMSO-d6) δ = 7.83 (d, 1H), 7.81 (d, 1H), 7.64 (d, 1H), 7.47 (dd, 1H), 6.21 (d, 1H), 3.87 (q, 2H), 2.25 (s, 3H), 1.97 - 1.92 (m, 1H), 1.11 (t, 3H), 0.97 - 0.87 (m, 2H), 0.73 - 0.67 (m, 2H) |
| 10 | 2-(3,4-dichlorophenyl)-1-ethyl-5-(3-fluoropyrazol-1-yl)-6-methyl-4-oxo-pyridine-3-carboxylic acid | 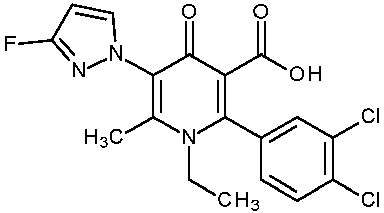 ¹H NMR (500 MHz, DMSO-d6) δ = 7.84 (d, 1H), 7.81 (d, 1H), 7.76 (t, 1H), 7.48 (dd, 1H), 6.27 (dd, 1H), 3.95 - 3.79 (m, 2H), 2.31 (s, 3H), 1.11 (t, 3H) |
| 11 | 2-(3,4-dichlorophenyl)-1-ethyl-5-[3-(methoxymethyl)pyrazol-1-yl]-6-methyl-4-oxo-pyridine-3-carboxylic acid | 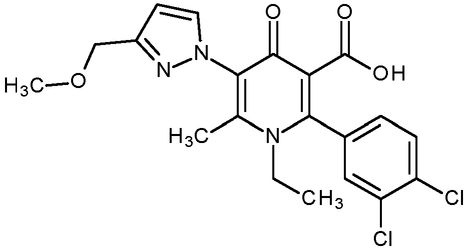 1H NMR (500 MHz, DMSO-d6) δ = 7.84 (d, 1H), 7.81 (d, 1H), 7.79 - 7.76 (m, 1H), 7.48 (dd, 1H), 6.49 (d, 1H), 4.43 (s, 2H), 3.88 (q, 2H), 3.29 (s, 3H), 2.25 (s, 3H), 1.12 (t, 3H) |
| 12 | 5-(3-acetylpyrazol-1-yl)-2-(3,4-dichlorophenyl)-1-ethyl-6-methyl-4-oxo-pyridine-3-carboxylic acid | 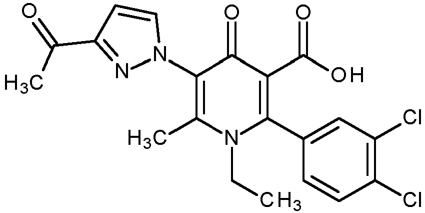 ¹H NMR (500 MHz, DMSO-d6) δ = 7.95 (d, 1H), 7.85 (d, 1H), 7.83 (d, 1H), 7.50 (dd, 1H), 6.94 (d, 1H), 3.89 (q, 2H), 2.53 - 2.51 (m, 3H), 2.26 (s, 3H), 1.13 (t, 3H) |
| 13 | 2-(3,4-dichlorophenyl)-5-(3-ethoxypyrazol-1-yl)-1-ethyl-6-methyl-4-oxo-pyridine-3-carboxylic acid | 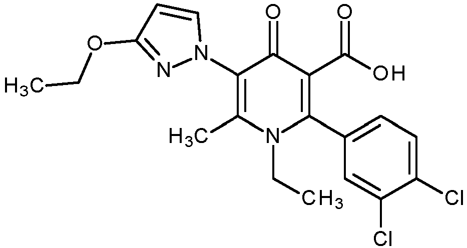 ¹H NMR (500 MHz, DMSO-d6) δ = 7.83 (d, 1H), 7.81 (d, 1H), 7.61 (d, 1H), 7.47 (dd, 1H), 5.94 (d, 1H), 4.14 (q, 2H), 3.87 (q, 2H), 2.32 (s, 3H), 1.32 (t, 3H), 1.11 (t, 3H) |
| 14 | 2-(3,4-difluorophenyl)-1-ethyl-6-methyl-4-oxo-5-[3-(trifluoromethyl)pyrazol-1-yl]pyridine-3-carboxylic acid | 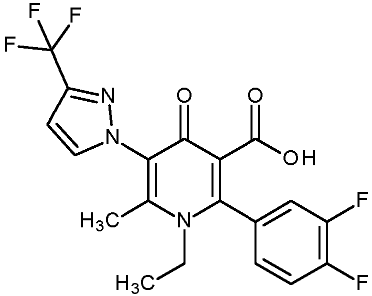 ¹H NMR (400 MHz, chloroform) δ = 7.81 (s, 1H), 7.36 (td, 1H), 7.13 (ddd, 1H), 7.01 (ddd, 1H), 6.80 (d, 1H), 3.97 (q, 2H), 2.43 (s, 3H), 1.28 (t, 3H) |
| 15 | 2-(3,4-dichlorophenyl)-1-ethyl-5-(3-isopropylpyrazol-1-yl)-6-methyl-4-oxo-pyridine-3-carboxylic acid | 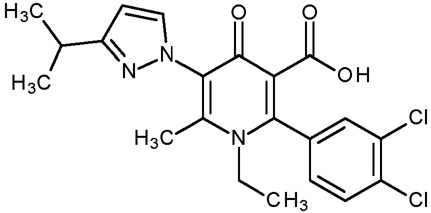 ¹H NMR (500 MHz, DMSO-d6) δ = 7.83 (d, 1H), 7.81 (d, 1H), 7.68 (d, 1H), 7.48 (dd, 1H), 6.35 (d, 1H), 3.88 (q, 2H), 3.00 - 2.93 (m, 1H), 2.25 (s, 3H), 1.25 (d, 6H), 1.18 - 1.06 (m, 3H) |
| 16 | 2-(3,4-dichlorophenyl)-1-ethyl-5-(6-fluoro-3-methyl-indazol-1-yl)-6-methyl-4-oxo-pyridine-3-carboxylic acid | 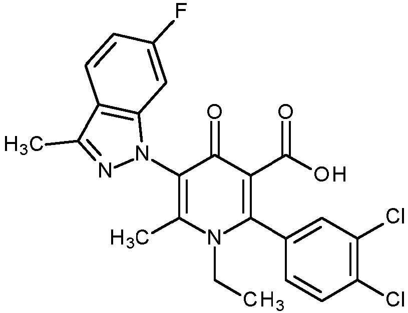 ¹H NMR (500 MHz, DMSO-d6) δ = 7.92 - 7.82 (m, 2.5H), 7.77 (d, 0.5H), 7.57 (dd, 0.5H), 7.45 (dd, 0.5H), 7.15 - 7.04 (m, 1.5H), 7.01 (dd, 0.5H), 3.98 - 3.86 (m, 2H), 2.57 (s, 3H), 2.27 (s, 3H), 1.16 (t, 3H) |
| 17 | 2-(3,4-dichlorophenyl)-1-ethyl-5-(4-methoxyindazol-1-yl)-6-methyl-4-oxo-pyridine-3-carboxylic acid | 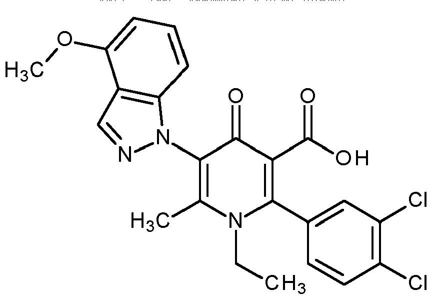 ¹H NMR (500 MHz, DMSO-d6) δ = 8.34 (s, 1H), 7.92 (d, 0.5H), 7.87 - 7.82 (m, 1.5H), 7.58 (dd, 0.5H), 7.52 (dd, 0.5H), 7.35 (dt, 1H), 6.84 (d, 0.5H), 6.80 (d, 0.5H), 6.70 (d, 1H), 3.98 (s, 3H), 3.92 (q, 2H), 2.20 (s, 3H), 1.15 (t, 3H) |
| 18 | 2-(3,4-dichlorophenyl)-1-ethyl-5-(5-fluoroindazol-1-yl)-6-methyl-4-oxo-pyridine-3-carboxylic acid | 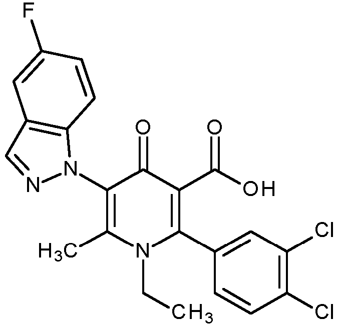 ¹H NMR (500 MHz, DMSO-d6) δ = 8.38 (s, 1H), 7.93 (d, 0.5H), 7.87 - 7.81 (m, 1.5H), 7.70 - 7.65 (m, 1H), 7.58 (dd, 0.5H), 7.50 (dd, 0.5H), 7.38 - 7.32 (m, 1.5H), 7.29 - 7.27 (m, 0.5H), 3.92 (q, 2H), 2.23 (s, 3H), 1.16 (t, 3H) |
| 19 | 2-(3,4-dichlorophenyl)-5-[3-(2,2-difluoroethoxy)pyrazol-1-yl]-1-ethyl-6-methyl-4-oxo-pyridine-3-carboxylic acid | 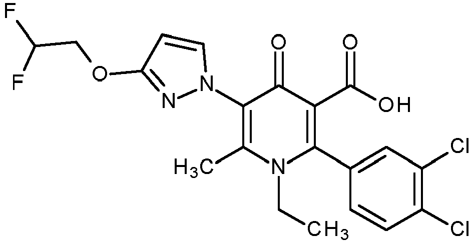 ¹H NMR (500 MHz, DMSO-d6) δ = 7.83 (d, 1H), 7.82 - 7.78 (m, 1H), 7.67 (d, 1H), 7.48 (dd, 1H), 6.50 - 6.27 (m, 1H), 6.06 (d, 1H), 4.40 (dt, 2H), 3.87 (q, 2H), 2.32 (s, 3H), 1.12 (brt, 3H) |
| 20 | 5-(3-cyanopyrazol-1-yl)-2-(3,4-dichlorophenyl)-1-ethyl-6-methyl-4-oxo-pyridine-3-carboxylic acid | 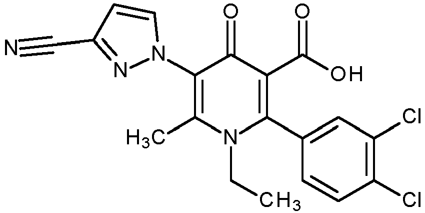 ¹H NMR (500 MHz, DMSO-d6) δ = 8.14 (d, 1H), 7.85 (d, 1H), 7.83 (d, 1H), 7.50 (dd, 1H), 7.24 (d, 1H), 3.87 (q, 2H), 2.23 (s, 3H), 1.12 (t, 3H) |
| 21 | 2-(3,4-dichlorophenyl)-1-ethyl-5-(6-methoxyindazol-1-yl)-6-methyl-4-oxo-pyridine-3-carboxylic acid | 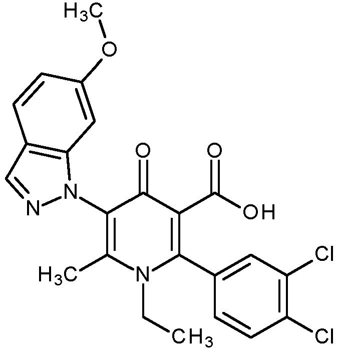 ¹H NMR (500 MHz, DMSO-d6) δ = 8.25 (s, 1H), 7.93 (d, 0.5H), 7.85 (d, 0.5H), 7.84 - 7.83 (m, 1H), 7.74 (dd, 1H), 7.58 (dd, 0.5H), 7.51 (dd, 0.5H), 6.89 (td, 1H), 6.70 (s, 0.5H), 6.64 (s, 0.5H), 3.96 - 3.92 (m, 2H), 3.79 (s, 3H), 2.23 (s, 3H), 1.17 (t, 3H) |
| 22 | 2-(3,4-dichlorophenyl)-1-ethyl-5-(7-fluoroindazol-2-yl)-6-methyl-4-oxo-pyridine-3-carboxylic acid | 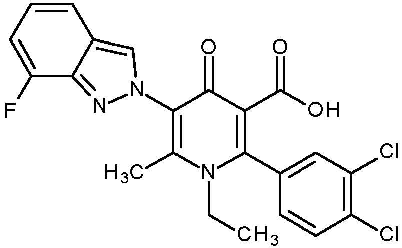 ¹H NMR (500 MHz, DMSO-d6) δ = 8.61 (d, 1H), 7.89 (d, 1H), 7.85 (d, 1H), 7.65 (d, 1H), 7.53 (dd, 1H), 7.15 - 7.07 (m, 2H), 3.96 - 3.89 (m, 2H), 2.25 (s, 3H), 1.15 (t, 3H) |
| 23 | 5-(3-chloro-1,2,4-triazol-1-yl)-2-(3,4-dichlorophenyl)-1-ethyl-6-methyl-4-oxo-pyridine-3-carboxylic acid | 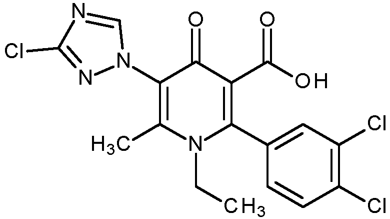 ¹H NMR (400 MHz, chloroform) δ = 8.35 (s, 1H), 7.64 (d, 1H), 7.38 (d, 1H), 7.14 (dd, 1H), 3.98 (q, 2H), 2.54 (s, 3H), 1.28 (t, 3H) |

### Biological examples

Seeds of a variety of test species are sown in standard soil in pots (*Ipomoea hederacea (IPOHE), Setaria faberi* (SETFA), *Zea mays (ZEAMX), Echinochloa crus-galli* (ECHCG), *Amaranthus palmeri* (AMAPA), *Amaranthus retoflexus* (AMARE)). After 8 days cultivation under controlled conditions in a glasshouse (at 24 °C /16 °C, day/night; 14 hours light; 65 % humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 1000 g/ha unless otherwise stated. The test plants are then grown in a glasshouse under controlled conditions in a glasshouse (at 24 °C/16 °C, day/night; 14 hours light; 65 % humidity) and watered twice daily. After 13 days the test is evaluated for the percentage damage caused to the plant. The biological activities are shown in the following table on a five-point scale (5 = 81-100%; 4 = 61-80%; 3=41-60%; 2=21-40%; 1=0-20%; - = not tested).

**TABLE B1: Pre-emergence Test**

| **Compound No.** | **IPOHE** | **SEFTA** | **ZEAMX** | **ECHCG** | **AMAPA** | **AMARE** |
|---|---|---|---|---|---|---|
| 1 | 4 | 1 | 0 | 0 | 0 | 0 |
| 2 | 4 | 3 | 1 | 0 | - | - |
| 3* | 0 | 2 | 0 | 1 | 0 | 0 |

| | | | | | | |
|---|---|---|---|---|---|---|
| ***data at 250g/Ha** | | | | | | |

**TABLE B2: Post-emergence Test**

| **Compound No.** | **IPOHE** | **SEFTA** | **ZEAMX** | **ECHCG** | **AMAPA** | **AMARE** |
|---|---|---|---|---|---|---|
| 1 | 2 | 1 | 1 | 1 | 3 | 2 |
| 2 | 4 | 4 | 3 | 4 | - | - |
| 3* | 2 | 3 | 2 | 3 | 3 | 3 |

| | | | | | | |
|---|---|---|---|---|---|---|
| ***data at 250g/Ha** | | | | | | |

## Claims

1. A compound of Formula (I): 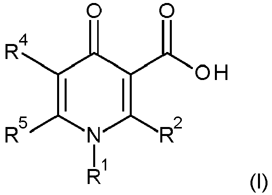 wherein
R¹ is C₁-C₆alkyl, C₁-C₆alkoxy, C₂-C₆alkenyl, C₂-C₆alkynyl, C₁-C₆alkoxyC₁-C₆alkyl, or C₃-C₆cycloalkyl;
R² is phenyl or heteroaryl, wherein the heteroaryl moiety is a 5- or 6-membered aromatic ring which comprises 1, 2, 3 or 4 heteroatoms individually selected from N, O and S, and wherein each phenyl and heteroaryl moiety may be optionally substituted with 1, 2, 3, or 4 groups, which may be the same or different, represented by R⁷;
R⁴ is heteroaryl wherein the heteroaryl moiety is a 5-membered aromatic monocyclic ring comprising 1, 2, or 3 nitrogen atoms, and wherein the heteroaryl moieties are attached to the rest of the molecule through a nitrogen atom in the heteroaryl ring, and wherein the heteroaryl moieties may each be optionally substituted with 1, 2, 3, or 4 groups, which may be the same or different, represented by R⁹;
R⁵ is hydrogen, halogen, C₁-C₆alkyl, C₁-C₆haloalkyl, or C₃-C₆cycloalkyl;
R⁷ is cyano, nitro, halogen, C₁-C₆alkyl, C₁-C₆alkoxy, C₁-C₆haloalkyl, C₁-C₆haloalkoxy, C₁-C₆alkoxyC₁-C₆alkyl, C₁-C₆alkylsulfanyl, C₁-C₆alkylsulfinyl, C₁-C₆alkylsulfonyl, C₁-C₆alkylsulfonamido, C₁-C₆alkylcarbonyl, C₁-C₆alkoxycarbonyl, C₁-C₆alkylaminocarbonyl, C₃-C₆cycloalkyl, C₃-C₆cycloalkylaminocarbonyl, or N,N-di(C₁-C₄alkyl)aminocarbonyl;
R⁹ is formyl, cyano, nitro, hydroxy, halogen, C₁-C₆alkyl, C₁-C₆alkoxy, C₁-C₆haloalkyl, C₁-C₆haloalkoxy, C₂-C₆alkenyloxy, cyanoC₁-C₆alkoxy, C₁-C₆alkoxyC₁-C₆alkyl, C₁-C₆alkoxyC₁-C₆alkoxy, C₁-C₆alkoxycarbonylC₁-C₆alkoxy, C₁-C₆alkoxycarbonyl, C₁-C₆alkylsulfanyl, C₁-C₆alkylsulfinyl, C₁-C₆alkylsulfonyl, C₁-C₆alkylsulfonamido, C₁-C₆alkylcarbonyl, C₁-C₆alkylaminocarbonyl, C₁-C₆alkylcarbonylamino, C₁-C₆alkylamino, C₁-C₆alkoxyC₁-C₆alkylaminocarbonyl, C₁-C₆alkoxyC₁-C₆alkylcarbonylamino, C₂-C₆alkenylcarbonylamino, C₃-C₆cycloalkyl, C₃-C₆cycloalkylaminocarbonyl, N,N-di(C₁-C₄alkyl)amino, N,N-di(C₁-C₄alkyl)aminocarbonyl, or benzyloxy;
wherein any two adjacent R⁹ groups together with the carbon atoms to which they are attached, may form a C₃-C₆cycloalkyl ring or phenyl ring, wherein the C₃-C₆cycloalkyl and phenyl moieties may be optionally substituted with 1, 2, 3 or 4 groups, which may be the same or different, represented by R¹⁰; or
any two adjacent R⁹ groups together with the carbon atoms to which they are attached, may form a 5- or 6-membered heteroaryl ring, wherein the heteroaryl moiety is a 5- or 6-membered aromatic ring which comprises 1, 2, 3 or 4 heteroatoms individually selected from N, O and S, and wherein the heteroaryl moiety may be optionally substituted with 1, 2, 3 or 4 groups, which may be the same or different, represented by R¹⁰; or
any two adjacent R⁹ groups together with the carbon atoms to which they are attached, may form a 5- or 6-membered heterocyclyl ring, wherein the heterocyclyl moiety is a 5- or 6-membered comprising 1 or 2 heteroatoms selected from O and N, and wherein the heterocyclyl ring may be optionally substituted with 1, 2, 3 or 4 groups, which may be the same or different, represented by R¹⁰;
R¹⁰ is halogen, C₁-C₃alkyl, or C₁-C₃alkoxy;
or a salt or an N-oxide thereof.

2. The compound according to claim 1, wherein R² is phenyl or heteroaryl, wherein the heteroaryl moiety is a 5- or 6-membered aromatic ring which comprises 1 or 2 heteroatoms individually selected from N and O, and wherein each phenyl and heteroaryl moiety may be optionally substituted with 1, 2, or 3 groups, which may be the same or different, represented by R⁷.

3. The compound according to claim 1 or claim 2, wherein R² is phenyl optionally substituted with 1 or 2 groups, which may be the same or different, represented by R⁷.

4. The compound according to any one of claims 1 to 3, wherein R⁵ is hydrogen or C₁-C₃alkyl.

5. The compound according to any one of claims 1 to 4, wherein R⁷ is cyano, nitro, halogen, C₁-C₃alkyl, C₁-C₃alkoxy, C₁-C₃haloalkyl, C₁-C₃haloalkoxy, C₁-C₃alkoxyC₁-C₃alkyl, C₁-C₃alkylcarbonyl, or C₃-C₆cycloalkyl.

6. The compound according to any one of claims 1 to 5, wherein R⁴ is heteroaryl wherein the heteroaryl moiety is a 5-membered aromatic monocyclic ring comprising 1, 2, or 3 nitrogen atoms, and wherein the heteroaryl moieties are attached to the rest of the molecule through a nitrogen atom in the heteroaryl ring, and wherein the heteroaryl moieties may each be optionally substituted with 1, 2, or 3 groups, which may be the same or different, represented by R⁹.

7. The compound according to any one of claims 1 to 6, wherein R⁴ is heteroaryl wherein the heteroaryl moiety is a 5-membered aromatic monocyclic ring comprising 2 or 3 nitrogen atoms, and wherein the heteroaryl moieties are attached to the rest of the molecule through a nitrogen atom in the heteroaryl ring, and wherein the heteroaryl moieties may each be optionally substituted with 1, 2, or 3 groups, which may be the same or different, represented by R⁹.

8. The compound according to any one of claims 1 to 7 wherein R⁹ is formyl, cyano, nitro, hydroxy, halogen, C₁-C₆alkyl, C₁-C₆alkoxy, C₁-C₆haloalkyl, C₁-C₆haloalkoxy, C₂-C₆alkenyloxy, cyanoC₁-C₄alkoxy, C₁-C₄alkoxyC₁-C₄alkyl, C₁-C₆alkoxyC₁-C₆alkoxy, C₁-C₄alkoxycarbonylC₁-C₄alkoxy, C₁-C₆alkoxycarbonyl, C₁-C₄alkylsulfanyl, C₁-C₄alkylsulfinyl, C₁-C₄alkylsulfonyl, C₁-C₄alkylsulfonamido, C₁-C₆alkylcarbonyl, C₁-C₆alkylaminocarbonyl, C₁-C₆alkylcarbonylamino, C₁-C₆alkylamino, C₁-C₆alkoxyC₁-C₆alkylaminocarbonyl, C₁-C₄alkoxyC₁-C₄alkylcarbonylamino, C₂-C₆alkenylcarbonylamino, C₃-C₆cycloalkyl, C₃-C₆cycloalkylaminocarbonyl, N,N-di(C₁-C₃alkyl)amino, N,N-di(C₁-C₃alkyl)aminocarbonyl, or benzyloxy; or
any two adjacent R⁹ groups together with the carbon atoms to which they are attached, may form a C₃-C₆cycloalkyl ring or phenyl ring, wherein the C₃-C₆cycloalkyl and phenyl moieties may be optionally substituted with 1, 2, or 3 groups, which may be the same or different, represented by R¹⁰; or
any two adjacent R⁹ groups together with the carbon atoms to which they are attached, may form a 5- or 6-membered heteroaryl ring, wherein the heteroaryl moiety is a 5- or 6-membered aromatic ring which comprises 1, 2, or 3 heteroatoms individually selected from N, O and S, and wherein the heteroaryl moiety may be optionally substituted with 1, 2, or 3 groups, which may be the same or different, represented by R¹⁰; or
any two adjacent R⁹ groups together with the carbon atoms to which they are attached, may form a 5- or 6-membered heterocyclyl ring, wherein the heterocyclyl moiety is a 5- or 6-membered comprising 1 or 2 heteroatoms selected from O and N, and wherein the heterocyclyl ring may be optionally substituted with 1, 2, or 3 groups, which may be the same or different, represented by R¹⁰.

9. The compound according to any one of claims 1 to 7, wherein R⁹ is formyl, cyano, nitro, halogen, C₁-C₄alkyl, C₁-C₃alkoxy, C₁-C₃haloalkyl, C₁-C₂haloalkoxy, C₂-C₃alkenyloxy, cyanoC₁-C₂alkoxy, C₁-C₃alkoxyC₁-C₃alkyl, C₁-C₃alkoxyC₁-C₃alkoxy, C₁-C₃alkoxycarbonylC₁-C₃alkoxy, C₁-C₃alkoxycarbonyl, C₁-C₃alkylsulfanyl, C₁-C₃alkylsulfonyl, C₁-C₃alkylcarbonyl, C₁-C₃alkylaminocarbonyl, C₁-C₃alkylcarbonylamino, C₁-C₃alkylamino, C₁-C₃alkoxyC₁-C₃alkylaminocarbonyl, C₁-C₂alkoxyC₁-C₂alkylcarbonylamino, C₃-C₄alkenylcarbonylamino, C₃-C₄cycloalkyl, or N,N-di(C₁-C₃alkyl)amino; or
any two adjacent R⁹ groups together with the carbon atoms to which they are attached, may form a C₅-C₆cycloalkyl ring or phenyl ring, wherein the C₅-C₆cycloalkyl and phenyl moieties may be optionally substituted with a single R¹⁰ group; or
any two adjacent R⁹ groups together with the carbon atoms to which they are attached, may form a 6-membered heteroaryl ring, wherein the heteroaryl moiety is a 6-membered aromatic ring which comprises a single nitrogen atom, and wherein the heteroaryl moiety may be optionally substituted with a single R¹⁰ group; or
any two adjacent R⁹ groups together with the carbon atoms to which they are attached, may form a 5- or 6-membered heterocyclyl ring, wherein the heterocyclyl moiety is a 5- or 6-membered comprising 1 or 2 heteroatoms selected from O and N, and wherein the heterocyclyl ring may be optionally substituted with a single R¹⁰ group.

10. The compound according to any one of claims 1 to 9, wherein R¹⁰ is halogen or C₁-C₃alkoxy.

11. A herbicidal composition comprising a compound according to any one of the previous claims and an agriculturally acceptable formulation adjuvant.

12. A herbicidal composition according to claim 11, further comprising at least one additional pesticide.

13. A herbicidal composition according to claim 12, wherein the additional pesticide is a herbicide or herbicide safener.

14. A method of controlling unwanted plant growth, comprising applying a compound of Formula (I) as defined in any one of claims 1 to 10, or a herbicidal composition according to any one of claims 11 to 13, to the unwanted plants or to the locus thereof.

15. Use of a compound of Formula (I) according to any one of claims 1 to 10 as a herbicide.

## Patentansprüche

1. Verbindung der Formel (I): 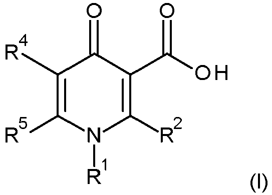 wobei
R¹ für C₁-C₆-Alkyl, C₁-C₆-Alkoxy, C₂-C₆-Alkenyl, C₂-C₆-Alkinyl, C₁-C₆-Alkoxy-C₁-C₆-alkyl oder C₃-C₆-Cycloalkyl steht;
R² für Phenyl oder Heteroaryl steht, wobei es sich bei der Heteroarylgruppierung um einen 5- oder 6-gliedrigen aromatischen Ring, der 1, 2, 3 oder 4 Heteroatome, die individuell aus N, O und S ausgewählt sind, umfasst, handelt und wobei die Phenyl- und Heteroarylgruppierung jeweils gegebenenfalls durch 1, 2, 3 oder 4 durch R⁷ wiedergegebene Gruppen, die gleich oder verschieden sein können, substituiert sein können;
R⁴ für Heteroaryl steht, wobei es sich bei der Heteroarylgruppierung um einen 5-gliedrigen aromatischen monocyclischen Ring, der 1, 2 oder 3 Stickstoffatome umfasst, handelt und wobei die Heteroarylgruppierungen über ein Stickstoffatom in dem Heteroarylring an den Rest des Moleküls gebunden sind und wobei die Heteroarylgruppierungen jeweils gegebenenfalls durch 1, 2, 3 oder 4 durch R⁹ wiedergegebene Gruppen, die gleich oder verschieden sein können, substituiert sein können;
R⁵ für Wasserstoff, Halogen, C₁-C₆-Alkyl, C₁-C₆-Halogenalkyl oder C₃-C₆-Cycloalkyl steht;
R⁷ für Cyano, Nitro, Halogen, C₁-C₆-Alkyl, C₁-C₆-Alkoxy, C₁-C₆-Halogenalkyl, C₁-C₆-Halogenalkoxy, C₁-C₆-Alkoxy-C₁-C₆-alkyl, C₁-C₆-Alkylsulfanyl, C₁-C₆-Alkylsulfinyl, C₁-C₆-Alkylsulfonyl, C₁-C₆-Alkylsulfonamido, C₁-C₆-Alkylcarbonyl, C₁-C₆-Alkoxycarbonyl, C₁-C₆-Alkylaminocarbonyl, C₃-C₆-Cycloalkyl, C₃-C₆-Cycloalkylaminocarbonyl oder N,N-Di(C₁-C₄-alkyl)aminocarbonyl steht;
R⁹ für Formyl, Cyano, Nitro, Hydroxy, Halogen, C₁-C₆-Alkyl, C₁-C₆-Alkoxy, C₁-C₆-Halogenalkyl, C₁-C₆-Halogenalkoxy, C₂-C₆-Alkenyloxy, Cyano-C₁-C₆C-alkoxy, C₁-C₆-Alkoxy-C₁-C₆-alkyl, C₁-C₆-Alkoxy-C₁-C₆-alkoxy, C₁-C₆-Alkoxycarbonyl-C₁-C₆-alkoxy, C₁-C₆-Alkoxycarbonyl, C₁-C₆-Alkylsulfanyl, C₁-C₆-Alkylsulfinyl, C₁-C₆-Alkylsulfonyl, C₁-C₆-Alkylsulfonamido, C₁-C₆-Alkylcarbonyl, C₁-C₆-Alkylaminocarbonyl, C₁-C₆-Alkylcarbonylamino, C₁-C₆-Alkylamino, C₁-C₆-Alkoxy-C₁-C₆-alkylaminocarbonyl, C₁-C₆-Alkoxy-C₁-C₆-alkylcarbonylamino, C₂-C₆-Alkenylcarbonylamino, C₃-C₆-Cycloalkyl, C₃-C₆-Cycloalkylaminocarbonyl, N,N-Di(C₁-C₄-alkyl)amino, N,N-Di(C₁-C₄-alkyl)aminocarbonyl oder Benzyloxy steht;
wobei beliebige zwei benachbarte R⁹-Gruppen zusammen mit den Kohlenstoffatomen, an die sie gebunden sind, einen C₃-C₆-Cycloalkylring oder Phenylring bilden können, wobei die C₃-C₆-Cycloalkyl- und Phenylgruppierungen gegebenenfalls durch 1, 2, 3 oder 4 durch R¹⁰ wiedergegebene Gruppen, die gleich oder verschieden sein können, substituiert sein können; oder
beliebige zwei benachbarte R⁹-Gruppen zusammen mit den Kohlenstoffatomen, an die sie gebunden sind, einen 5- oder 6-gliedrigen Heteroarylring bilden können, wobei es sich bei der Heteroarylgruppierung um einen 5- oder 6-gliedrigen aromatischen Ring, der 1, 2, 3 oder 4 Heteroatome, die individuell aus N, O und S ausgewählt sind, umfasst, handelt und wobei die Heteroarylgruppierung gegebenenfalls durch 1, 2, 3 oder 4 durch R¹⁰ wiedergegebene Gruppen, die gleich oder verschieden sein können, substituiert sein kann; oder beliebige zwei benachbarte R⁹-Gruppen zusammen mit den Kohlenstoffatomen, an die sie gebunden sind, einen 5- oder 6-gliedrigen Heterocyclylring bilden können, wobei die Heterocyclylgruppierung 5- oder 6-gliedrig ist und 1 oder 2 Heteroatome, die aus O und N ausgewählt sind, umfasst und wobei der Heterocyclylring gegebenenfalls durch 1, 2, 3 oder 4 durch R¹⁰ wiedergegebene Gruppen, die gleich oder verschieden sein können, substituiert sein kann;
R¹⁰ für Halogen, C₁₋C₃-Alkyl oder C₁-C₃-Alkoxy steht; oder ein Salz oder ein N-Oxid davon.

2. Verbindung nach Anspruch 1, wobei R² für Phenyl oder Heteroaryl steht, wobei es sich bei der Heteroarylgruppierung um einen 5- oder 6-gliedrigen aromatischen Ring, der 1 oder 2 Heteroatome, die individuell aus N und O ausgewählt sind, umfasst, handelt und wobei die Phenyl- und Heteroarylgruppierung jeweils gegebenenfalls durch 1, 2 oder 3 durch R⁷ wiedergegebene Gruppen, die gleich oder verschieden sein können, substituiert sein können.

3. Verbindung nach Anspruch 1 oder Anspruch 2, wobei R² für Phenyl, das gegebenenfalls durch 1 oder 2 durch R⁷ wiedergegebene Gruppen, die gleich oder verschieden sein können, substituiert ist, steht.

4. Verbindung nach einem der Ansprüche 1 bis 3, wobei R⁵ für Wasserstoff oder C₁-C₃-Alkyl steht.

5. Verbindung nach einem der Ansprüche 1 bis 4, wobei R⁷ für Cyano, Nitro, Halogen, C₁-C₃-Alkyl, C₁-C₃-Alkoxy, C₁-C₃-Halogenalkyl, C₁-C₃-Halogenalkoxy, C₁-C₃-Alkoxy-C₁-C₃-alkyl, C₁-C₃-Alkylcarbonyl oder C₃-C₆-Cycloalkyl steht.

6. Verbindung nach einem der Ansprüche 1 bis 5, wobei R⁴ für Heteroaryl steht, wobei es sich bei der Heteroarylgruppierung um einen 5-gliedrigen aromatischen monocyclischen Ring, der 1, 2 oder 3 Stickstoffatome umfasst, handelt und wobei die Heteroarylgruppierungen über ein Stickstoffatom in dem Heteroarylring an den Rest des Moleküls gebunden sind und wobei die Heteroarylgruppierungen jeweils gegebenenfalls durch 1, 2 oder 3 durch R⁹ wiedergegebene Gruppen, die gleich oder verschieden sein können, substituiert sein können.

7. Verbindung nach einem der Ansprüche 1 bis 6, wobei R⁴ für Heteroaryl steht, wobei es sich bei der Heteroarylgruppierung um einen 5-gliedrigen aromatischen monocyclischen Ring, der 2 oder 3 Stickstoffatome umfasst, handelt und wobei die Heteroarylgruppierungen über ein Stickstoffatom in dem Heteroarylring an den Rest des Moleküls gebunden sind und wobei die Heteroarylgruppierungen jeweils gegebenenfalls durch 1, 2 oder 3 durch R⁹ wiedergegebene Gruppen, die gleich oder verschieden sein können, substituiert sein können.

8. Verbindung nach einem der Ansprüche 1 bis 7, wobei R⁹ für Formyl, Cyano, Nitro, Hydroxy, Halogen, C₁-C₆-Alkyl, C₁-C₆-Alkoxy, C₁-C₆-Halogenalkyl, C₁-C₆-Halogenalkoxy, C₂-C₆-Alkenyloxy, Cyano-C₁-C₄-alkoxy, C₁-C₄-Alkoxy-C₁-C₄-alkyl, C₁-C₆-Alkoxy-C₁-C₆-alkoxy, C₁-C₄-Alkoxycarbonyl-C₁-C₄-alkoxy, C₁-C₆-Alkoxycarbonyl, C₁-C₄-Alkylsulfanyl, C₁-C₄-Alkylsulfinyl, C₁-C₄-Alkylsulfonyl, C₁-C₄-Alkylsulfonamido, C₁-C₆-Alkylcarbonyl, C₁-C₆-Alkylaminocarbonyl, C₁-C₆-Alkylcarbonylamino, C₁-C₆-Alkylamino, C₁-C₆-Alkoxy-C₁-C₆-alkylaminocarbonyl, C₁-C₄-Alkoxy-C₁-C₄-alkylcarbonylamino, C₂-C₆-Alkenylcarbonylamino, C₃-C₆-Cycloalkyl, C₃-C₆-Cycloalkylaminocarbonyl, N,N-Di(C₁-C₃-alkyl)amino, N,N-Di(C₁-C₃-alkyl)aminocarbonyl oder Benzyloxy steht; oder beliebige zwei benachbarte R⁹-Gruppen zusammen mit den Kohlenstoffatomen, an die sie gebunden sind, einen C₃-C₆-Cycloalkylring oder Phenylring bilden können, wobei die C₃-C₆-Cycloalkyl- und Phenylgruppierungen gegebenenfalls durch 1, 2 oder 3 durch R¹⁰ wiedergegebene Gruppen, die gleich oder verschieden sein können, substituiert sein können; oder
beliebige zwei benachbarte R⁹-Gruppen zusammen mit den Kohlenstoffatomen, an die sie gebunden sind, einen 5- oder 6-gliedrigen Heteroarylring bilden können, wobei es sich bei der Heteroarylgruppierung um einen 5- oder 6-gliedrigen aromatischen Ring, der 1, 2 oder 3 Heteroatome, die individuell aus N, O und S ausgewählt sind, umfasst, handelt und wobei die Heteroarylgruppierung gegebenenfalls durch 1, 2 oder 3 durch R¹⁰ wiedergegebene Gruppen, die gleich oder verschieden sein können, substituiert sein kann; oder beliebige zwei benachbarte R⁹-Gruppen zusammen mit den Kohlenstoffatomen, an die sie gebunden sind, einen 5- oder 6-gliedrigen Heterocyclylring bilden können, wobei die Heterocyclylgruppierung 5- oder 6-gliedrig ist und 1 oder 2 Heteroatome, die aus O und N ausgewählt sind, umfasst und wobei der Heterocyclylring gegebenenfalls durch 1, 2 oder 3 durch R¹⁰ wiedergegebene Gruppen, die gleich oder verschieden sein können, substituiert sein kann.

9. Verbindung nach einem der Ansprüche 1 bis 7, wobei R⁹ für Formyl, Cyano, Nitro, Halogen, C₁-C₄-Alkyl, C₁-C₃-Alkoxy, C₁-C₃-Halogenalkyl, C₁-C₂-Halogenalkoxy, C₂-C₃-Alkenyloxy, Cyano-C₁-C₂-alkoxy, C₁-C₃-Alkoxy-C₁-C₃-alkyl, C₁-C₃-Alkoxy-C₁-C₃-alkoxy, C₁-C₃-Alkoxycarbonyl-C₁-C₃-alkoxy, C₁-C₃-Alkoxycarbonyl, C₁-C₃-Alkylsulfanyl, C₁-C₃-Alkylsulfonyl, C₁-C₃-Alkylcarbonyl, C₁-C₃-Alkylaminocarbonyl, C₁-C₃-Alkylcarbonylamino, C₁-C₃-Alkylamino, C₁-C₃-Alkoxy-C₁-C₃-alkylaminocarbonyl, C₁-C₂-Alkoxy-C₁-C₂-alkylcarbonylamino, C₃-C₄-Alkenylcarbonylamino, C₃-C₄-Cycloalkyl oder N,N-Di(C₁-C₃-alkyl)amino steht; oder
beliebige zwei benachbarte R⁹-Gruppen zusammen mit den Kohlenstoffatomen, an die sie gebunden sind, einen C₅-C₆-Cycloalkylring oder Phenylring bilden können, wobei die C₅-C₆-Cycloalkyl- und Phenylgruppierungen gegebenenfalls durch eine einzige R¹⁰-Gruppe substituiert sein können; oder
beliebige zwei benachbarte R⁹-Gruppen zusammen mit den Kohlenstoffatomen, an die sie gebunden sind, einen 6-gliedrigen Heteroarylring bilden können, wobei es sich bei der Heteroarylgruppierung um einen 6-gliedrigen aromatischen Ring, der ein einziges Stickstoffatom umfasst, handelt und wobei die Heteroarylgruppierung gegebenenfalls durch eine einzige R¹⁰-Gruppe substituiert sein kann; oder
beliebige zwei benachbarte R⁹-Gruppen zusammen mit den Kohlenstoffatomen, an die sie gebunden sind, einen 5- oder 6-gliedrigen Heterocyclylring bilden können, wobei die Heterocyclylgruppierung 5- oder 6-gliedrig ist und 1 oder 2 Heteroatome, die aus O und N ausgewählt sind, umfasst und wobei der Heterocyclylring gegebenenfalls durch eine einzige R¹⁰-Gruppe substituiert sein kann.

10. Verbindung nach einem der Ansprüche 1 bis 9, wobei R¹⁰ für Halogen oder C₁-C₃-Alkoxy steht.

11. Herbizide Zusammensetzung, umfassend eine Verbindung nach einem der vorhergehenden Ansprüche und ein landwirtschaftlich unbedenkliches Formulierungshilfsmittel.

12. Herbizide Zusammensetzung nach Anspruch 11, ferner umfassend mindestens ein zusätzliches Pestizid.

13. Herbizide Zusammensetzung nach Anspruch 12, wobei es sich bei dem zusätzlichen Pestizid um ein Herbizid oder einen Herbizid-Safener handelt.

14. Verfahren zur Bekämpfung von unerwünschtem Pflanzenwuchs, bei dem man eine wie in einem der Ansprüche 1 bis 10 definierte Verbindung der Formel (I) oder eine herbizide Zusammensetzung nach einem der Ansprüche 11 bis 13 auf die unerwünschten Pflanzen oder deren Standort ausbringt.

15. Verwendung einer Verbindung der Formel (I) nach einem der Ansprüche 1 bis 10 als Herbizid.

## Revendications

1. Composé de formule (I) : 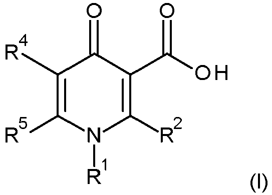
R¹ étant C₁-C₆alkyle, C₁-C₆alcoxy, C₂-C₆alcényle, C₂-C₆alcynyle, C₁-C₆alcoxyC₁-C₆alkyle ou C₃-C₆cycloalkyle ; R² étant phényle ou hétéroaryle, le groupement hétéroaryle étant un cycle aromatique à 5 ou 6 chaînons qui comprend 1, 2, 3 ou 4 hétéroatomes individuellement choisis parmi N, O et S, et chaque groupement phényle et hétéroaryle pouvant éventuellement être substitué par 1, 2, 3 ou 4 groupes, qui peuvent être les mêmes ou différents, représentés par R⁷ ;
R⁴ étant hétéroaryle, le groupement hétéroaryle étant un cycle monocyclique aromatique à 5 chaînons comprenant 1, 2 ou 3 atomes d'azote, et les groupements hétéroaryle étant fixés au reste de la molécule par l'intermédiaire d'un atome d'azote dans le cycle hétéroaryle, et les groupements hétéroaryle pouvant chacun être éventuellement substitués par 1, 2, 3 ou 4 groupes, qui peuvent être les mêmes ou différents, représentés par R⁹ ;
R⁵ étant hydrogène, halogène, C₁-C₆alkyle, C₁-C₆halogénoalkyle ou C₃-C₆cycloalkyle ;
R⁷ étant cyano, nitro, halogène, C₁-C₆alkyle, C₁-C₆alcoxy, C₁-C₆halogénoalkyle, C₁-C₆halogénoalcoxy, C₁-C₆alcoxyC₁-C₆alkyle, C₁-C₆alkylsulfanyle, C₁-C₆alkylsulfinyle, C₁-C₆alkylsulfonyle, C₁-C₆alkylsulfonamido, C₁-C₆alkylcarbonyle, C₁-C₆alcoxycarbonyle, C₁-C₆alkylaminocarbonyle, C₃-C₆cycloalkyle, C₃-C₆cycloalkylaminocarbonyle ou N,N-di(C₁-C₄alkyl)aminocarbonyle ;
R⁹ étant formyle, cyano, nitro, hydroxy, halogène, C₁-C₆alkyle, C₁-C₆alcoxy, C₁-C₆halogénoalkyle, C₁-C₆halogénoalcoxy, C₂-C₆alcényloxy, cyanoC₁-C₆alcoxy, C₁-C₆alcoxyC₁-C₆alkyle, C₁-C₆alcoxyC₁-C₆alcoxy, C₁-C₆alcoxycarbonylC₁-C₆alcoxy, C₁-C₆alcoxycarbonyle, C₁-C₆alkylsulfanyle, C₁-C₆alkylsulfinyle, C₁-C₆alkylsulfonyle, C₁-C₆alkylsulfonamido, C₁-C₆alkylcarbonyle, C₁-C₆alkylaminocarbonyle, C₁-C₆alkylcarbonylamino, C₁-C₆alkylamino, C₁-C₆alcoxyC₁-C₆alkylaminocarbonyle, C₁-C₆alcoxyC₁-C₆alkylcarbonylamino, C₂-C₆alcénylcarbonylamino, C₃-C₆cycloalkyle, C₃-C₆cycloalkylaminocarbonyle, N,N-di(C₁-C₄alkyl) amino, N,N-di(C₁-C₄alkyl)aminocarbonyle ou benzyloxy ;
deux groupes quelconques R⁹ adjacents conjointement avec les atomes de carbone auxquels ils sont fixés, pouvant former un cycle C₃-C₆cycloalkyle ou un cycle phényle, les groupements C₃-C₆cycloalkyle et phényle pouvant éventuellement être substitués par 1, 2, 3 ou 4 groupes, qui peuvent être les mêmes ou différents, représentés par R¹⁰ ; ou
deux groupes quelconques R⁹ adjacents conjointement avec les atomes de carbone auxquels ils sont fixés, pouvant former un cycle hétéroaryle à 5 ou 6 chaînons, le groupement hétéroaryle étant un cycle aromatique à 5 ou 6 chaînons qui comprend 1, 2, 3 ou 4 hétéroatomes individuellement choisis parmi N, O et S, et le groupement hétéroaryle pouvant éventuellement être substitué par 1, 2, 3 ou 4 groupes, qui peuvent être les mêmes ou différents, représentés par R¹⁰ ; ou
deux groupes quelconques R⁹ adjacents conjointement avec les atomes de carbone auxquels ils sont fixés, pouvant former un cycle hétérocyclyle à 5 ou 6 chaînons, le groupement hétérocyclyle étant un cycle 5 ou 6 chaînons comprenant 1 ou 2 hétéroatomes choisis parmi O et N, et le cycle hétérocyclyle pouvant éventuellement être substitué par 1, 2, 3 ou 4 groupes, qui peuvent être les mêmes ou différents, représentés par R¹⁰ ;
R¹⁰ étant halogène, C₁-C₃alkyle ou C₁-C₃alcoxy ;
ou sel ou N-oxyde correspondant.

2. Composé selon la revendication 1, R² étant phényle ou hétéroaryle, le groupement hétéroaryle étant un cycle aromatique à 5 ou 6 chaînons qui comprend 1 ou 2 hétéroatomes individuellement choisis parmi N et O, et chaque groupement phényle et hétéroaryle pouvant éventuellement être substitué par 1, 2 ou 3 groupes, qui peuvent être les mêmes ou différents, représentés par R⁷.

3. Composé selon la revendication 1 ou la revendication 2, R² étant phényle éventuellement substitué par 1 ou 2 groupes, qui peuvent être les mêmes ou différents, représentés par R⁷.

4. Composé selon l'une quelconque des revendications 1 à 3, R⁵ étant hydrogène ou C₁-C₃alkyle.

5. Composé selon l'une quelconque des revendications 1 à 4, R⁷ étant cyano, nitro, halogène, C₁-C₃alkyle, C₁-C₃alcoxy, C₁-C₃halogénoalkyle, C₁-C₃halogénoalcoxy, C₁-C₃alcoxyC₁-C₃alkyle, C₁-C₃alkylcarbonyle ou C₃-C₆cycloalkyle.

6. Composé selon l'une quelconque des revendications 1 à 5, R⁴ étant hétéroaryle, le groupement hétéroaryle étant un cycle monocyclique aromatique à 5 chaînons comprenant 1, 2 ou 3 atomes d'azote, et les groupements hétéroaryle étant fixés au reste de la molécule par l'intermédiaire d'un atome d'azote dans le cycle hétéroaryle, et les groupements hétéroaryle pouvant chacun être éventuellement substitués par 1, 2 ou 3 groupes, qui peuvent être les mêmes ou différents, représentés par R⁹.

7. Composé selon l'une quelconque des revendications 1 à 6, R⁴ étant hétéroaryle, le groupement hétéroaryle étant un cycle monocyclique aromatique à 5 chaînons comprenant 2 ou 3 atomes d'azote, et les groupements hétéroaryle étant fixés au reste de la molécule par l'intermédiaire d'un atome d'azote dans le cycle hétéroaryle, et les groupements hétéroaryle pouvant chacun être éventuellement substitués par 1, 2 ou 3 groupes, qui peuvent être les mêmes ou différents, représentés par R⁹.

8. Composé selon l'une quelconque des revendications 1 à 7, R⁹ étant formyle, cyano, nitro, hydroxy, halogène, C₁-C₆alkyle, C₁-C₆alcoxy, C₁-C₆halogénoalkyle, C₁-C₆halogénoalcoxy, C₂-C₆alcényloxy, cyanoC₁-C₄alcoxy, C₁-C₄alcoxyC₁-C₄alkyle, C₁-C₆alcoxyC₁-C₆alcoxy, C₁-C₄alcoxycarbonylC₁-C₄alcoxy, C₁-C₆alcoxycarbonyle, C₁-C₄alkylsulfanyle, C₁-C₄alkylsulfinyle, C₁-C₄alkylsulfonyle, C₁-C₄alkylsulfonamido, C₁-C₆alkylcarbonyle, C₁-C₆alkylaminocarbonyle, C₁-C₆alkylcarbonylamino, C₁-C₆alkylamino, C₁-C₆alcoxyC₁-C₆alkylaminocarbonyle, C₁-C₄alcoxyC₁-C₄alkylcarbonylamino, C₂-C₆alcénylcarbonylamino, C₃-C₆cycloalkyle, C₃-C₆cycloalkylaminocarbonyle, N,N-di(C₁-C₃alkyl)amino, N,N-di(C₁-C₃alkyl)aminocarbonyle, ou benzyloxy ; ou
deux groupes quelconques R⁹ adjacents conjointement avec les atomes de carbone auxquels ils sont fixés, pouvant former un cycle C₃-C₆cycloalkyle ou un cycle phényle, les groupements C₃-C₆cycloalkyle et phényle pouvant éventuellement être substitués par 1, 2, ou 3 groupes, qui peuvent être les mêmes ou différents, représentés par R¹⁰ ; ou
deux groupes quelconques R⁹ adjacents conjointement avec les atomes de carbone auxquels ils sont fixés, pouvant former un cycle hétéroaryle à 5 ou 6 chaînons, le groupement hétéroaryle étant un cycle aromatique à 5 ou 6 chaînons qui comprend 1, 2 ou 3 hétéroatomes individuellement choisis parmi N, O et S, et le groupement hétéroaryle pouvant éventuellement être substitué par 1, 2 ou 3 groups, qui peuvent être les mêmes ou différents, représentés par R¹⁰ ; ou
deux groupes quelconques R⁹ adjacents conjointement avec les atomes de carbone auxquels ils sont fixés, pouvant former un cycle hétérocyclyle à 5 ou 6 chaînons, le groupement hétérocyclyle étant un cycle 5 ou 6 chaînons comprenant 1 ou 2 hétéroatomes choisis parmi O et N, et le cycle hétérocyclyle pouvant éventuellement être substitué par 1, 2 ou 3 groupes, qui peuvent être les mêmes ou différents, représentés par R¹⁰.

9. Composé selon l'une quelconque des revendications 1 à 7, R⁹ étant formyle, cyano, nitro, halogène, C₁-C₄alkyle, C₁-C₃alcoxy, C₁-C₃halogénoalkyle, C₁-C₂halogénoalcoxy, C₂-C₃alcényloxy, cyanoC₁-C₂alcoxy, C₁-C₃alcoxyC₁-C₃alkyle, C₁-C₃alcoxyC₁-C₃alcoxy, C₁-C₃alcoxycarbonylC₁-C₃alcoxy, C₁-C₃alcoxycarbonyle, C₁-C₃alkylsulfanyle, C₁-C₃alkylsulfonyle, C₁-C₃alkylcarbonyle, C₁-C₃alkylaminocarbonyle, C₁-C₃alkylcarbonylamino, C₁-C₃alkylamino, C₁-C₃alcoxyC₁-C₃alkylaminocarbonyle, C₁-C₂alcoxyC₁-C₂alkylcarbonylamino, C₃-C₄alcénylcarbonylamino, C₃-C₄cycloalkyle ou N,N-di(C₁-C₃alkyl)amino ; ou
deux groupes quelconques R⁹ adjacents conjointement avec les atomes de carbone auxquels ils sont fixés, pouvant former un cycle C₅-C₆cycloalkyle ou un cycle phényle, les groupements C₅-C₆cycloalkyle et phényle pouvant éventuellement être substitués par un seul groupe R¹⁰ ; ou
deux groupes quelconques R⁹ adjacents conjointement avec les atomes de carbone auxquels ils sont fixés, pouvant former un cycle hétéroaryle à 6 chaînons, le groupement hétéroaryle étant un cycle aromatique à 6 chaînons qui comprend un seul atome d'azote, et le groupement hétéroaryle pouvant éventuellement être substitué par un seul groupe R¹⁰ ; ou
deux groupes quelconques R⁹ adjacents conjointement avec les atomes de carbone auxquels ils sont fixés, pouvant former un cycle hétérocyclyle à 5 ou 6 chaînons, le groupement hétérocyclyle étant un cycle 5 ou 6 chaînons comprenant 1 ou 2 hétéroatomes choisis parmi O et N, et le cycle hétérocyclyle pouvant éventuellement être substitué par un seul groupe R¹⁰.

10. Composé selon l'une quelconque des revendications 1 à 9, R¹⁰ étant halogène ou C₁-C₃alcoxy.

11. Composition herbicide comprenant un composé selon l'une quelconque des revendications précédentes et un adjuvant de formulation acceptable sur le plan agricole.

12. Composition herbicide selon la revendication 11, comprenant en outre au moins un pesticide supplémentaire.

13. Composition herbicide selon la revendication 12, dans laquelle le pesticide supplémentaire est un herbicide ou un phytoprotecteur vis-à-vis des herbicides.

14. Procédé de lutte contre une croissance végétale non souhaitée, comprenant l'application d'un composé de formule (I) tel que défini dans l'une quelconque des revendications 1 à 10, ou d'une composition herbicide selon l'une quelconque des revendications 11 à 13, sur les végétaux non souhaités ou sur le lieu où ils se développent.

15. Utilisation d'un composé de formule (I) selon l'une quelconque des revendications 1 à 10 en tant qu'herbicide.
